(19) Europäisches Patentamt
European Patent Office
Office européen des brevets

(11) Veröffentlichungsnummer: **0 513 543 B1**

(12) # EUROPÄISCHE PATENTSCHRIFT

(45) Veröffentlichungstag der Patentschrift: **25.10.95**

(51) Int. Cl.$^{6}$: **C07K 5/06**, A61K 38/05

(21) Anmeldenummer: **92106584.3**

(22) Anmeldetag: **16.04.92**

(54) **Amidinophenylalaninderivate, Verfahren zu deren Herstellung, deren Verwendung und diese enthaltende Mittel als Antikoagulantien.**

(30) Priorität: **11.05.91 DE 4115468**

(43) Veröffentlichungstag der Anmeldung:
**19.11.92 Patentblatt 92/47**

(45) Bekanntmachung des Hinweises auf die Patenterteilung:
**25.10.95 Patentblatt 95/43**

(84) Benannte Vertragsstaaten:
**AT BE CH DE DK ES FR GB GR IT LI LU NL PT SE**

(56) Entgegenhaltungen:
**EP-A- 0 236 163**
**EP-A- 0 236 164**

**CHEMICAL ABSTRACTS, Band 110, Nr. 11, 13. MUrz 1989, Columbus, Ohio, USA B. VOIGT et al. "Synthesis of N-alpha-(arylsulfonyl)-4- -amidinophenyl-**

**alanylprolines and N-alpha-(arylsulfonyl- glycyl)-4-amidinophenyl- - alanylprolines and their testing as inhibitors of serine proteases. Part 33: Synthetic inhibitors of serine proteases" Seite 760, Spalte 2, Zusammenfassung- Nr. 95 774g**

(73) Patentinhaber: **BEHRINGWERKE Aktiengesellschaft**
**Postfach 1140**
**D-35001 Marburg (DE)**

(72) Erfinder: **Stüber, Werner**
**Am Pfahltor 5**
**W-3531 Lahntal 3 (DE)**
Erfinder: **Koschinsky, Rainer**
**Am Heydwolf 18**
**W-3575 Kirchhain (DE)**
Erfinder: **Dickneite, Gerhard**
**Zum Neuen Hieb 31**
**W-3550 Marburg (DE)**
Erfinder: **Cenek Kolar**
**Deutschhausstrasse 20**
**3550 Marburg (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

CHEMICAL ABSTRACTS, Band 110, Nr. 7, 13. Februar 1989, Columbus, Ohio, USA H.P. KLOECKING et al. "Influence of alpha-NAPAP in thrombin-induced release of plasminogen activator" Seite 38, Spalte 2, Zusammenfassung-Nr. 50 941t

CHEMICAL ABSTRACTS, Band 111, Nr. 9, 28. August 1989, Columbus, Ohio, USA J. HAUPTMANN et al. "Pharma- cological characterization of a new structural variant of a 4-amidinophenylalanine amide-type synthetic thrombin inhibitor" Seite 46, Spalte 1, Zusammenfassung-Nr. 70 596h

**Beschreibung**

Die Erfindung betrifft Amidinophenylalaninderivate, die Synthese dieser Verbindungen, deren Verwendung sowie pharmazeutische Mittel, die diese Verbindungen enthalten.

Bekanntlich führen eine Reihe von pathophysiologischen Bedingungen zu einem Verbrauch von Antithrombin III (AT III), dem wichtigsten Thrombininhibitor im Plasma. Ein Abfall von AT III führt zu einem erhöhten Thromboserisiko, wie man u.a. auch von Fällen mit angeborenem Mangel an AT III weiß. Ein Abfall auf Werte unterhalb 75% der Norm hat thromboembolische Komplikationen zur Folge. Diese Komplikationen treten häufig in Form einer disseminierten intravasalen Gerinnung nach Operationen und bei Schockzuständen auf. In vielen Fällen treten dabei lebensbedrohliche Blutgerinnsel auf. Zur Therapie und Prophylaxe thrombotischer Erkrankungen werden bisher in der Medizin Antikoagulantien mit unterschiedlicher Wirkungsweise eingesetzt. Zur akuten Bekämpfung eines Thromboserisikos werden Substanzen wie AT III, Heparin und neuerdings auch Hirudin eingesetzt. Langzeitprophylaxen werden mit Cumarin- und Indandionderivaten ausgeführt. Die aufgeführten Antikoagulantien sind jedoch mit z.T. erheblichen Nachteilen behaftet.

Heparin beispielsweise kann aufgrund seiner Polysaccharidstruktur nur parenteral appliziert werden und seine Wirkung ist auch auf einen funktionsfähigen Antithrombin III-Spiegel angewiesen. Cumarine wirken direkt der Proteinbiosynthese entgegen, indem die Vitamin K-abhängigen Gerinnungsfaktoren II, VII, IX und X nicht mehr ausreichend zur Verfügung gestellt werden können und somit das Gerinnungspotential reduziert wird. Daraus ergibt sich eine zeitliche Verzögerung der Wirksamkeit. Als bekannte Nebenwirkungen gelten hämorrhagische Hautnekrosen, Nausea und Haarausfall.

Demgegenüber haben niedermolekulare Thrombininhibitoren den Vorteil, daß sie kofaktorenunabhängig direkt auf Thrombin einwirken, indem sie direkt an das aktive Zentrum binden und damit das Enzym gleichsam verschliessen. Aufgrund ihrer chemischen Struktur können diese Substanzen potentiell oral appliziert werden.

Besondere Bekanntheit haben Aminosäurederivate auf Argininbasis und Amidinophenylalaninbasis erlangt. Zur ersten Gruppe zählen Verbindungen wie D-Phenylalanyl-L-prolyl-argininaldehydund (2R,4R)-4-Methyl-1-[$N^2$-(3-methyl-1,2,3,4-tetrahydro-8-chinolinsulfonyl)-L-arginyl]-2-piperidincarbonsäure monohydrat ("MD 805"). MD 805 ist ein kompetitiver spezifischer Thrombininhibitor, der auch therapeutisch eingesetzt wird. Ein weiteres bekanntes Amidinophenylalaninderivat ist das beta-Naphthylsulfonyl-glycyl-R,S-4-amidinophenylalanylpiperidid (NAPAP). In EP 0236 163 und in EP 0236 164 sind Derivate des NAPAPs beschrieben. Dabei wurde Glycin ($NH_2$-$CH_2$-COOH) durch eine andere Aminosäure der Struktur ($NH_2$-$CHR_1$-COOH), worin R1 eine niedere Alkylgruppe, eine niedere Hydroxyalkylgruppe, eine Phenylgruppe oder eine 4-Hydroxyphenylgruppe ist, ersetzt. Das 4-Amidinophenylalanin (Aph) kann zu N-Methyl-Aph methyliert sein.

Außerdem wurden für NAPAP verschiedene Derivatisierungen am Arylsulfonyl, "Brücken"-Glycin und am Piperidinring beschrieben. Am geeignetsten sind demnach alpha- oder beta-Naphthyl-sulfonylgruppen am N-Terminus, wogegen Heteroaryl-sulfonyl-gruppen wie 8-Chinolinsulfonyl um Zehnerpotenzen schlechter sind. Ein weiterer Nachteil dieser Verbindungen, der besonders die NAPAP-Strukturen betrifft, liegt in deren geringer Verträglichkeit, dem ungünstigen pharmakokinetischen Verhalten sowie zum Teil einer zu geringen Spezifität, wobei eine zu hohe Antitrypsinwirksamkeit besonders zu erwähnen ist. Einer oralen Anwendung von Substanzen, die para-Amidinophenylalanin enthalten, steht die Tatsache entgegen, daß die Stabilität gegen Darmenzyme und Leberenzyme nicht optimal ist, so daß die Substanzen im Darm und in der Leber zu schnell metabolisiert werden. Durch Ersatz des Piperidins in NAPAP durch ein Prolin kann eine verbesserte Verträglichkeit und eine günstigere Pharmakokinetik erreicht werden. Ein ganz entscheidender Nachteil der Prolinverbindung ist allerdings ein unerwünschter Wirkungsverlust bezüglich der Thrombininhibition um den Faktor 100 gegenüber NAPAP. Darüberhinaus gibt es bis heute keine Möglichkeit, die Spezifität dieser Verbindungen gegenüber Thrombin und Trypsin durch Einführung von Substituenten signifikant zu verbessern.

Aufgabe der Erfindung war es deshalb, neue Verbindungen auf Basis von Amidinophenylalanin bereitzustellen, die den bekannten Verbindungen aufgrund ihrer antithrombotischen Wirksamkeit überlegen sind, eine hohe enzymatische Resistenz, eine verbesserte Verträglichkeit, eine verbesserte Spezifität und eine verbesserte Pharmakokinetik aufweisen.

Gegenstand dieser Erfindung sind demnach Verbindungen der Formel I

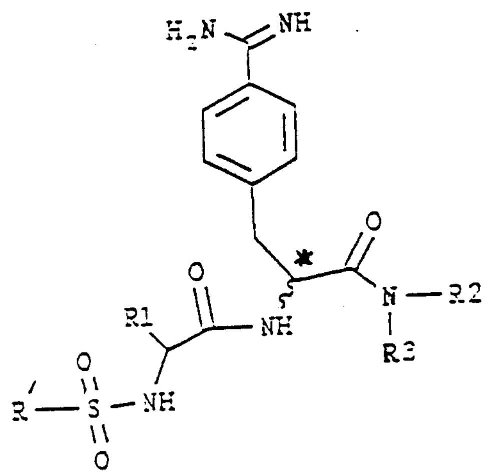

I

worin

R' ein in der alpha- oder beta-Position gebundener Naphthalinring ist, der gegebenenfalls mit Alkylgruppen, die bis zu 3 C-Atome enthalten, und/oder Alkoxygruppen mit jeweils bis zu 3 C-Atomen, derivatisiert ist, oder ein in der alpha- oder beta-Position gebundener Tetralinring oder Indanring ist, der gegebenenfalls mit Alkylgruppen, die aus bis zu 3 C-Atomen bestehen, und/oder auch Alkoxygruppen mit jeweils bis zu 3 C-Atomen derivatisiert ist,
oder ein Phenylring ist, der gegebenenfalls mit Alkylgruppen, die bis zu 4 C-Atome enthalten, und/oder mit bis zu drei Gruppen der Struktur O-X , in der O Sauerstoff und X Wasserstoff, Methyl, Ethyl, n-Propyl, i-Propyl oder tert.-Butyl ist, und/oder mit einer Gruppe der Struktur -COOY, in der Y Wasserstoff, Methyl, Ethyl, n-Propyl, i-Propyl, tert.-Butyl, i-Butyl, i-Pentyl oder neo-Pentyl ist, derivatisiert ist,
oder ein Chromansystem ist, das vorzugsweise mit bis zu 5 Alkylgruppen, die bis zu 3 C-Atome enthalten, derivatisiert ist,

$R_1$ eine Gruppe der Struktur A-B ist, wobei A = $-(CH_2)_n-$ und n = 1-4 und B eine Säurefunktion ist, ausgewählt aus der Gruppe Carboxylfunktion, die gegebenenfalls verestert sein kann oder als Amid vorliegt, wobei die Ester einen Alkohol mit bis zu 17 C-Atomen enthalten, Sulfonsäurefunktion, eine Funktion einer Säure des Phosphors, eine Boronsäurefunktion und Tetrazolgruppe, oder $R_1$ eine Gruppe der Struktur A-B-C ist, wobei A obige Bedeutung hat, B Carbonyl oder Sulfonyl ist und die Gruppe C sich von einer N-gebundenen alpha, beta, gamma oder delta Aminosäure oder der Gruppe der N-glycosidisch verknüpften Uronsäuren ableitet, und

$R_2$ und $R_3$ gleich oder verschieden sein können und Alkylgruppen mit bis zu 4 C-Atomen sind oder zusammen einen heterocyclischen Ring mit bis zu 8 Ringgliedern bilden, der mit einer Hydroxygruppe oder einer Hydroxyalkygruppe mit bis zu 3 C-Atomen derivatisiert sein kann und diese Hydroxygruppe gegebenenfalls verestert vorliegt, wobei die entsprechenden Säuren Carbonsäuren sind, die bis zu 17 C-Atome enthalten, und in der das mit * gezeichnete C-Atom in der R oder S Struktur, vorzugsweise aber in der R-Struktur vorliegt.

Diese neuartigen Verbindungen sind dadurch gekennzeichnet, daß die Aminosäure $R_1$ in Formel I eine saure Aminosäure ist. Saure Aminosäuren sind beispielsweise die in Proteinen vorkommenden Aminosäuren Glutaminsäure und Asparaginsäure oder auch Cysteinsäure, jedoch auch unnatürliche Aminosäuren wie die Aminoadipinsäure oder 3-Phosphonoalanin oder die 2-Amino-3-boropropionsäure. Da diese Aminosäuren in Folge eines unsymmetrisch substituierten C-Atoms als chirale Substanzen vorliegen, zeigen die mit diesen Aminosäuren hergestellten neuartigen Verbindungen auch eine unterschiedliche Wirksamkeit, wobei überwiegend die entsprechenden Aminosäuren in der L-Form zu Endverbindungen mit einer höheren Wirksamkeit führen.

Die in der Struktur $R_1$ genannte Gruppierung A zeichnet sich bevorzugt dadurch aus, daß n die Zahl 1 oder 2 annimmt, wobei ganz besonders die Zahl 1 bevorzugt wird. Ist die Gruppe C eine Aminosäure, so ist

damit vorzugsweise eine alpha, beta, gamma oder delta-Aminosäure gemeint. Solche Aminosäuren sind beispielsweise alpha-Aminoadipinsäure, alpha-Aminobuttersäure, gamma-Aminobuttersäure, 4-Aminobenzoesäure, 2-Aminobenzoesäure, epsilon-Aminocapronsäure, 1-Aminocyclohexancarbonsäure, 2-Aminocyclohexancarbonsäure, 3-Aminocyclohexancarbonsäure, 4-Aminocyclohexancarbonsäure, 1-Aminocyclopentancarbonsäure, 2-Amino-4,5-dimethylphenoxazon(3)-1,8-dicarbonsäure, 2-Amino-3-hydroxy-4-methylbenzoesäure, alpha-Amino- isobuttersäure, Aminohydroxybuttersäure, β-Aminoiso- buttersäure, Alanin, β-Alanin, Dehydroalanin, C-Allylglycin, Alliin, 2-Amino-3-methyl-butyl-1,3-thiazolin-5-carbonsäure, 6-Amino-penicillansäure, alpha-Amino-pimelinsäure, 1-Amino-cyclopropancarbonsäure, Asparagin, Asparaginsäure, alpha-Aminosuberinsäure, Azetidincarbonsäure, Aziridincarbonsäure, Baikiain, C-Benzylphenylalanin, Canavanin, Citrullin, Cystein, Cystathionin, Djenkolsäure, 3,4-Dihydroxyphenylalanin, 4-Sulfonylphenylalanin, 2,2-Dimethyl-1,3-thiazolidin-4-carbonsäure, Felinin, Glutamin, Glutaminsäure, Glycin, Hexahydronicotinsäure, Homo-Cystein, Histidin, Homoserin, delta-Hydroxy-Lysin, gamma-Hydroxyprolin, β-Hydroxyprolin, Isoleucin, Isoserin, Isovalin, Kynorenin, Lanthionin, Leucenin, Leucin, Lysergsäure, Methionin, Mimosin, Minalin, Norleucin, Norvalin, Pantonin, Pipecolinsäure, Penicillamin, Phenylalanin, C-Phenyl-Glycin, Picolinsäure, Prolin, Dehydroprolin, β-Phenyl-serin, Pyridyl-2-alanin, Pyrrolidon-(5)-carbonsäure-(2), (Pyrazolyl-3)-alanin, Chinoxalin-2-carbonsäure, Roseonin, Sarkosin, Selenocystein, Selenomethionin, Serin, Statin, (1,3-Thiazolyl-(2))-alanin, β-(1,3-Thiazolyl-(2))-alanin, Threonin, Thyronin, Thyroxin, 1,3-Thiazolin-2-carbonsäure, Tertiärleucin, Tryptophan, Tryptathionin, Tyrosin, Valin. Diese Aminosäuren können für den Fall, daß sie optisch aktiv sind, in der D- oder L-Form vorkommen.

Für den Fall, daß C eine Aminodicarbonsäure ist, zu denen beispielsweise die D- oder L-Glutaminsäure oder Asparaginsäure zählt, kann eine dieser Carboxylgruppen derivatisiert sein, wobei bevorzugt die alpha Carboxylgruppe derivatisiert vorliegt. Solche Derivate sind vorzugsweise Amide, die sich beispielsweise von Ammoniak, Methylamin, Dimethylamin, Benzylamin, Piperidin oder Morpholin ableiten.

Ist die Gruppe C eine N-glycosidisch verknüpfte Uronsäure, so läßt sich eine derartige Verbindung durch die folgende allgemeine Formel darstellen:

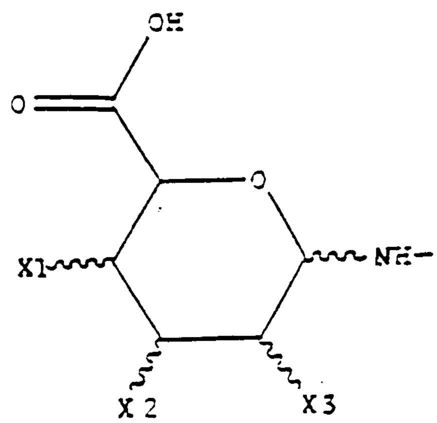

mit

X1 = -H, -OH, $-CH_3$, O-Acetyl
X2 = -H, -OH, $-CH_3$, O-Acetyl
X3 = -H, -OH, $-CH_3$, O-Acetyl

Überraschenderweise sind die neuen Verbindungen hochpotente Thrombininhibitoren. Überraschend ist auch, daß die Stabilität gegenüber Darm- und Leberhomogenaten sowie gegen Trypsin und Chymotrypsin gegenüber bekannten Verbindungen entscheidend verbessert ist.

Überraschenderweise wurde auch gefunden, daß die Wirksamkeit sowie die Spezifität der erfindungsgemäßen Verbindungen durch die Gruppierungen R' entscheidend beeinflußt wird. Die Verbindung mit R' = β-Naphthylsulfonyl und $R_1$ = Asp zeigt hinsichtlich der Thrombin- und Trypsinhemmung eine sehr ähnliche Wirksamkeit, also eine geringe Spezifität. Durch Einführung von Alkylgruppen und/oder Hydroxyalkylgruppen und/oder Hydroxygruppen und/oder Carboxylgruppen an dem Sulfonsäurerest R', zu denen vorzugsweise Gruppen mit bis zu 3 C-Atomen zählen, besonders bevorzugt Methyl- und/oder Methoxygruppen, kann eine bedeutende Spezifitätssteigerung gegen Thrombin erreicht werden. Das bedeutet, daß durch die Substitution mit einer oder mehreren solchen Gruppen Substanzeigenschaften bewerkstelligt wird, die sich insbesondere und überraschend in einer verstärkten Thrombinhemmung zeigten, wobei gleichzeitig Trypsin weniger gehemmt wurde.

Es können also die Substanzeigenschaften dieser als Thrombininhibitoren interessanten Substanzklasse durch die hier beschriebenen Derivatisierungsmuster in entscheidender Weise beeinflußt werden, die zu überlegenen Verbindungen führen.

Von besonderer Wirksamkeit, wie anhand der Ki-Werte erkennbar, sind die erfindungsgemäßen Strukturen auf Basis von R' = Phenyl und $R_1$ = $-CH_2-COOH$ (d.h. L-Asp) sowie der davon abgeleiteten Verbindungen, wobei R' entweder Alkylgruppen, die bis zu 4 C-Atome enthalten, oder auch Gruppen der Struktur O-X , in der O = Sauerstoff, und X = Wasserstoff, Methyl-, Ethyl-, n-Propyl, i-Propyl- oder tert.-Butyl-, oder auch eine Gruppe der Struktur -COOY , in der Y = Wasserstoff, Methyl-, Ethyl-, n-Propyl-, i-Propyl, tert.-Butyl-, i-Butyl-, i-Pentyl- oder neo-Pentyl- trägt.

Im näheren Sinne und ohne Beschränkung auf die nachfolgenden Beispiele, eignen sich als R' bevorzugt die folgenden Gruppen:

| | |
|---|---|
| 6,7-Dimethoxynaphthyl- | (β-Dmn) |
| 5-Methoxynaphthyl- | (β-Mns) |
| 2,2,5,7,8,-Pentamethylchroman- | (Pmc) |
| 5,6,7,8,-Tetrahydronaphthalin- | (Thn) |
| 5,6,7,8,-Tetramethylnaphthyl- | (Tmn) |
| Phenyl- | (Phl) |
| 4-Methoxy-2,3,6-trimethylphenyl- | (Mtr) |
| 2,3,4,5,6-Pentamethylphenyl- | (Pme) |
| 4-Methoxy-2,3,5,6-tetramethylphenyl- | (Mte) |
| 4-Hydroxy-2,3,6-trimethylphenyl- | (Htr) |
| 4-Carboxyphenyl- | (Cph). |

Von ganz besonderer Wirksamkeit zeichnete sich dabei die Mtr-Gruppe aus, d.h. es handelt sich dabei um eine Struktur, die sich von dem 4-Methoxy-2,3,6-Trimethylbenzolsulfonsäurechlorid ableitet.

Als Gruppe C eignen sich alpha-, beta-, gamma- oder delta-Aminosäuren und deren Derivate, sowie N-glycosidisch gebundene Uronsäure, wie beispielsweise die β-D-Aminoglucuronsäure.

Überraschenderweise sind die erfindungsgemäßen Verbindungen auch durch eine verbesserte Verträglichkeit gekennzeichnet und deshalb den Verbindungen nach dem Stand der Technik überlegen. So zum Beispiel wurde für die Verbindung β-Naphthylsulfonyl-L-Asp-D,L-Aph-Pip ein $LD_{50}$-Wert (Ratte, i.v.) von ca. 120 mg/kg bestimmt, wogegen dieser Wert beim NAPAP bei etwa 20 mg/kg liegt. Es wurde auch gefunden, daß eine weitere Carboxylgruppe an der Gruppe R' die Verträglichkeit der erfindungsgemäßen Verbindungen weiter verbessert.

Die verbesserte Verträglichkeit der erfindungsgemäßen Inhibitoren mit sauren Gruppen zeigt sich in einer verminderten Histaminfreisetzung und einem verminderten Blutdruckabfall.

Daß sich diese Verbindungen neben der starken Antithrombinwirksamkeit, Spezifität und günstigeren Verträglichkeit durch eine verbesserte Stabilität gegen Enzyme auszeichnen, beispielsweise gegen Trypsin und Chymotrypsin sowie Leber- und Darmhomogenaten, macht diese Substanzen zu einer Klasse interessanter Antikoagulantien.

Als weiterer Vorteil der erfindungsgemäßen Verbindungen ist die orale Bioverfügbarkeit zu erwähnen, die diese Verbindungen so besonders attraktiv macht.

Die erfindungsgemäßen Verbindungen können als Antithrombotika zur Therapie, um die Bildung von Blutgerinnseln zu verhindern, oder in der Diagnose eingesetzt werden.

Zum Gegenstand der Erfindung gehört auch die Bereitstellung von Derivaten dieser Substanzen, insbesondere Esterverbindungen. Diese sind dadurch gekennzeichnet, daß die Carboxygruppe an der Aminosäure $R_1$ mit aliphatischen Alkoholen mit bis zu 17 C- Atomen verestert ist. Solche Ester sind demnach abgeleitet von Alkoholen wie Methyl- , Ethyl-, Isopropyl-, n-Propyl, n-Butyl-, iso-Butyl-, tert.-Butyl-, n-Pentyl-, iso-Pentyl-, neo-Pentyl-, Octyl-, Decyl-, Dodecyl-, Hexadecyl- oder Heptadecylalkohol. Durch die Derivatisierung mit Alkoholen wird die Lipidlöslichkeit der erfindungsgemäßen Substanzen verbessert, was sich in einer günstigen enteralen Aufnahmefähigkeit widerspiegelt.

Sind an den Resten $R_2$ bzw. $R_3$ Hydroxylfunktionen vorhanden, so können diese gegebenenfalls mit Carbonsäuren verestert sein. Solche Carbonsäuren sind beispielsweise Essigsäure, Bernsteinsäure, Pivalinsäure, Hexancarbonsäure, Oktancarbonsäure, Dekancarbonsäure, Dodekancarbonsäure oder Hexadekancarbonsäure.

Da die Amidinofunktion auf Grund der basischen Wirkung in der Regel als Salz vorliegt, können die erfindungsgemäßen Thrombininhibitoren auch in unterschiedlichen Salzformen vorkommen. Die Salzform hat dabei einen wesentlichen Einfluß auf die Löslichkeit der Verbindungen sowie auf die Resorbierbarkeit im Falle der therapeutischen Anwendung. Als Salzformen seien hier genannt Formiate, Acetate, Caproate, Oleate oder Salze von Carbonsäuren mit bis zu 16 C-Atomen, Chloride, Bromide, Jodide, Alkansulfonate mit

bis zu 10 C-Atomen, Salze von Dicarbonsäuren und Tricarbonsäuren, wie Citrate oder Tartrate.

Bevorzugt sind besonders:

Eine Verbindung der Formel I, in der R' β-Naphthyl, $R_1$ $-CH_2-COOX$ mit X gleich Wasserstoff und $R_2$ und $R_3$ zusammen Piperidin sind.

Eine Verbindung der Formel I, in der R' β-6,7-Dimethoxynaphthyl-, $R_1$ $-CH_2-COOX$ mit X gleich Wasserstoff und $R_2$ und $R_3$ zusammen Piperidin sind.

Eine Verbindung der Formel I, in der R' β-Tetralin, $R_1$ $-CH_2-COOX$ mit X gleich Wasserstoff und $R_2$ und $R_3$ zusammen Piperidin sind.

Eine Verbindung der Formel I, in der R' 4-Methoxy-2,3,6-trimethylphenyl-, $R_1$ $-CH_2-COOX$ mit X gleich Wasserstoff und $R_2$ und $R_3$ zusammen Piperidin sind.

Eine Verbindung der Formel I, in der R' 4-Carboxyphenyl-, R1 -CH2-COOX mit X gleich Wasserstoff und R2 und R3 zusammen Piperidin sind.

Eine Verbindung der Formel I, in der R' 4-Hydroxy-2,3,6-trimethylphenyl-, $R_1$ $-CH_2-COOX$ mit X gleich Wasserstoff und $R_2$ und $R_3$ zusammen Piperidin sind.

Eine Verbindung der Formel I, in der R' 2,2,5,7,8-Pentamethylchroman-, $R_1$ $-CH_2-COOX$ mit X gleich Wasserstoff und $R_2$ und $R_3$ zusammen Piperidin sind.

Eine Verbindung der Formel I, dadurch gekennzeichnet, daß X einen Alkoholrest darstellt, der bis zu 17 C-Atome aufweist.

Eine Verbindung der Formel I, wobei $R_1$ die Struktur $-(CH_2)_n-SO_3H$ mit $n = 1$ bis 4 aufweist.

Eine Verbindung der Formel I, wobei $R_1$ die Struktur $-(CH_2)_n-PO(OH)_2$ mit $n = 1$ bis 4 aufweist.

Eine Verbindung der Formel I, dadurch gekennzeichnet, daß $R_2$ und $R_3$ zusammen 3-Hydroxymethylpiperidin sind.

Verbindung der Formel I, dadurch gekennzeichnet, daß die Hydroxyfunktion mit einer Carbonsäure verestert ist, die bis zu 17 C-Atome enthält.

Gegenstand der Erfindung ist auch ein Verfahren zur Herstellung einer Verbindung der Formel I, dadurch gekennzeichnet, daß eine geschützte saure Aminosäure über die alpha-ständige Carboxylgruppe an die alpha-Aminofunktion eines p-Cyanophenylalaninamidderivats gekoppelt wird, die N-alpha-Schutzgruppe entfernt wird, ein Sulfonylchlorid an diese Stickstoffgruppe gekoppelt wird, die Cyanogruppe in die Amidinofunktion überführt wird und gegebenenfalls eine vorhandene Schutzgruppe an der Drittfunktion der sauren Aminosäure abgespalten wird.

Zum Einbau der sauren Aminosäuren, zu denen insbesondere die Aminosäuren Asparaginsäure (Asp) oder Glutaminsäure (Glu) zählen, muß auf die Verwendung geschützter Aminosäuren zurückgegriffen werden. Obligatorisch ist dabei die Blockierung der N-alpha-Funktion mit einer Schutzgruppe. Als Schutzgruppen dienen dabei bevorzugt Gruppierungen vom Urethantyp wie tert.-Butyloxycarbonyl (Boc), Benzyloxycarbonyl (Z bzw. Cbo), Biphenylisopropyloxycarbonyl (Bpoc), Dimethoxydimethylbenzyloxycarbonyl (Ddz) oder 9-Fluorenylmethyloxycarbonyl (Fmoc). Besondere Beachtung muß dabei der Maskierung der Carboxylgruppe in der Seitenfunktion gewidmet werden.

Hierbei werden bevorzugt Ester eingesetzt, die je nach Bedarf nach der Synthese abgespalten werden können. Ist nach der Synthese eine Abspaltung der Seitengruppenmaskierung beabsichtigt, wird vorzugsweise der tert.- Butylester eingesetzt. Als N-alpha Schutzgruppe bewährte sich z.B. die basisch abspaltbare Fmoc-Gruppe oder die sehr säurelabile Bpoc- oder Ddz- Gruppe. Sollte die Seitenfunktion in der sauren Aminofunktion in der Endverbindung verestert bleiben, so wird vorzugsweise der entsprechende N-alpha-Z geschützte Aminosäure alpha tert.-Butylester mit dem gewünschten Alkohol verestert, die Z-Gruppe hydrogenolytisch entfernt, das entsprechende Sulfonylchlorid mit dem Aminosäurederivat umgesetzt, der tert.-Butylester acidolytisch abgespalten und das isolierte Produkt an die Aminofunktion des Cyanophenylalaninderivats gekoppelt. Generell erfolgt die Herstellung der Sulfamidbindung nach Standardverfahren, indem die entsprechenden Sulfonsäurechloride an die Aminogruppe der sauren Aminosäure gekoppelt wird. Die Herstellung von Verbindungen, die an R' eine Phenolgruppe enthalten erfolgt über die entsprechenden geschützten Phenolether, besonders bevorzugt tert.-Butylether, die nach Überführung in die Sulfonylchloride und Herstellung der Sulfamidbindung acidolytisch abgespalten werden und somit die gewünschten Endverbindungen liefern.

Freie Phenolgruppen lassen sich auch nach anderen bekannten Verfahren herstellen, so z.B. mit Demethylierungsmitteln, besonders bevorzugt Bortribromid in einem organischen Lösemittel, wobei bevorzugt Dichlormethan zu erwähnen ist.

Eine zweite Möglichkeit besteht darin, zuerst die Sulfamidbindung zur sauren Aminosäure herzustellen und diese daraufhin an das para-Cyanophenylalaninderivat zu kondensieren. Zur Herstellung der Arylsulfonylaminosäuren-tert.-butylester bzw. Heteroarylsulfonyl-aminosäuren-tert.-butylester wird das entsprechende Sulfonsäurechlorid und der Aminosäure-tert-butylester-alphamethylester in Dimethylformamid unter Basenzusatz, beispielsweise N-Methylmorpholin oder Diisopropylethylamin, umgesetzt. Der Methylester wird mittels Lauge verseift und es erfolgt die Ankopplung an das Aph- bzw. Cyanphenylalaninderivat mittels einer Carbodiimidreaktion. Die erfindungsgemäßen Verbindungen mit einer Sulfonsäuregruppe (B = $SO_3H$), vorzugsweise mit Cysteinsulfonsäure, konnten unter Verwendung von N-alpha geschützter Cysteinsulfonsäure hergestellt werden. Hierzu wird an den Stickstoff der Aminosäure die Boc-Gruppe gebunden und diese Verbindung an das Cyanophenylalaninpiperidid gekoppelt. Nach Abspaltung der Schutzgruppe wird das gewünschte Sulfonsäurechlorid damit in Reaktion gebracht. Geeignet ist auch, die entsprechende Cysteinverbindung mit freier Sulfhydrylfunktion herzustellen und diese dann mit einem Oxidationsmittel, beispielsweise mit Perameisensäure, in die Sulfonsäure zu überführen. Bevorzugt wird dabei Boc-Cys-(SStBu) oder Boc-Cys(Trt) benutzt. Die Oxidationsreaktionen werden nach Abspaltung der Schwefelschutzgruppen ausgeführt.

Erfindungsgemäße Verbindungen, mit einer Gruppe C aus der Gruppe der alpha-, beta-, gamma-, oder delta-Aminosäure werden nach an sich bekannten Verfahren hergestellt. Vorzugsweise wird eine N-geschützte saure Aminosäure, beispielsweise Asparaginsäure, wobei als Schutzgruppe bevorzugt die endgültige Sulfonamidstruktur eingeführt wird, mit einer carboxylgeschützten Aminosäure gekoppelt. Die alpha-Carboxylfunktion ist dabei geschützt, bevorzugt als Methylester. Die Ankopplung erfolgt vorzugsweise mit Kondensationsmitteln vom Carbodiimidtyp, vorzugsweise mit Dicyclohexylcarbodiimid. Hydroxybenzotriazol kann zu der Reaktion zugesetzt werden. Als Lösemittel wird bevorzugt Dimethylformamid benutzt.

Nach Isolation der Substanzen wird die alpha ständige Carboxylgruppe freigelegt, was im Falle der Methylester durch Verseifung geschieht.

Diese Vorstufen werden dann mit den bereits hier beschriebenen Verfahren auf die Aminofunktion des Cyanophenylalanins gekoppelt und zu weiteren Reaktion gebracht, d.h. die Amidinofunktion hergestellt. Zur Herstellung von erfindungsgemäßen Verbindungen, wobei die Gruppe C eine N-glycosidisch verknüpfte Uronsäure ist, wird zunächst die N-alpha und C-alpha geschützte saure Aminosäure mit dem entsprechend geschützten Kohlehydratteil verknüpft. Die Herstellung erfolgt nach an sich literaturbekannten Verfahren, wie z.B. A. Klemer et al,; J. Carbohydrate Chemistry, 7 (4), 785-797 (1988) oder T. Takeda et al.; Carbohydrate Research, 207, 71-79 (1990) oder L. Urge et al.; Tetrahedron Letters, 32(9), 3445-3448 (1991) oder R. Walczyna et al.; Carbohydrate Research; 180, 147-151 (1988). Nach Abspaltung der Stickstoffschutzgruppe der Aminosäure wird ein Sulfonsäurechlorid wie oben beschrieben angekoppelt und die C-alpha Carboxylgruppe abgespalten. Dann erfolgt die Kopplung an die Aminogruppe des C-terminalen Anteils, wozu bevorzugt ein Amidinophenylalaninamid, besonders bevorzugt D-4-Amidinophenylalanin-piperidid verwendet wird. Nach Entschützung der Uronsäure werden die Substanzen mit den üblichen Verfahren, wie Gelpermeationschromatographie oder Ionenaustauschchromatographie gereinigt.

Zur Bereitstellung der Amidinofunktion werden die p-Cyanophenylalaninverbindungen in Pyridin und Triethylamin gelöst und mit Schwefelwasserstoff gesättigt. Nach 2 bis 3 Tagen wird die Lösung in salzsaures Wasser eingerührt und der Niederschlag isoliert. Nach Methylierung mit einem Methylierungsmittel, vorzugsweise Methyljodid und Umsetzung mit einem Ammoniumsalz wie Ammoniumacetat in einem Alkohol, vorzugsweise Methanol, wird das Peptidderivat mit Amidinofunktion erhalten. Nach acidolytischer Behandlung mit Trifluoressigsäure oder HCl in Essigsäure wird das gewünschte Endprodukt erhalten. Die sich anschließende Gelpermeationschromatographie an $^{R}$Sephadex LH-20 in Methanol erbringt die reine Substanz. Die Endverbindungen werden mittels NMR und Massenspektrometrie auf Identität und mittels HPLC und Dünnschichtchromatographie auf Reinheit geprüft. Zur Überführung in die gewünschten Salzformen dient vorzugsweise die Ionenaustauschchromatographie. Dabei wird die entsprechende Verbindung an ein carboxymethyliertes Ionenaustauscherharz, z.B CM-$^{R}$Fractogel gebunden, und mit der gewünschten Säure eluiert. Das Endprodukt wird durch Lyophilisation in kristalliner Form gewonnen. Weitere gewünschte Salzformen können aus den Acetatsalzen gewonnen werden.

Gegenstand der Erfindung ist besonders auch ein Verfahren zur Herstellung einer Verbindung der Formel I, dadurch gekennzeichnet, daß man eine Verbindung der Formel II

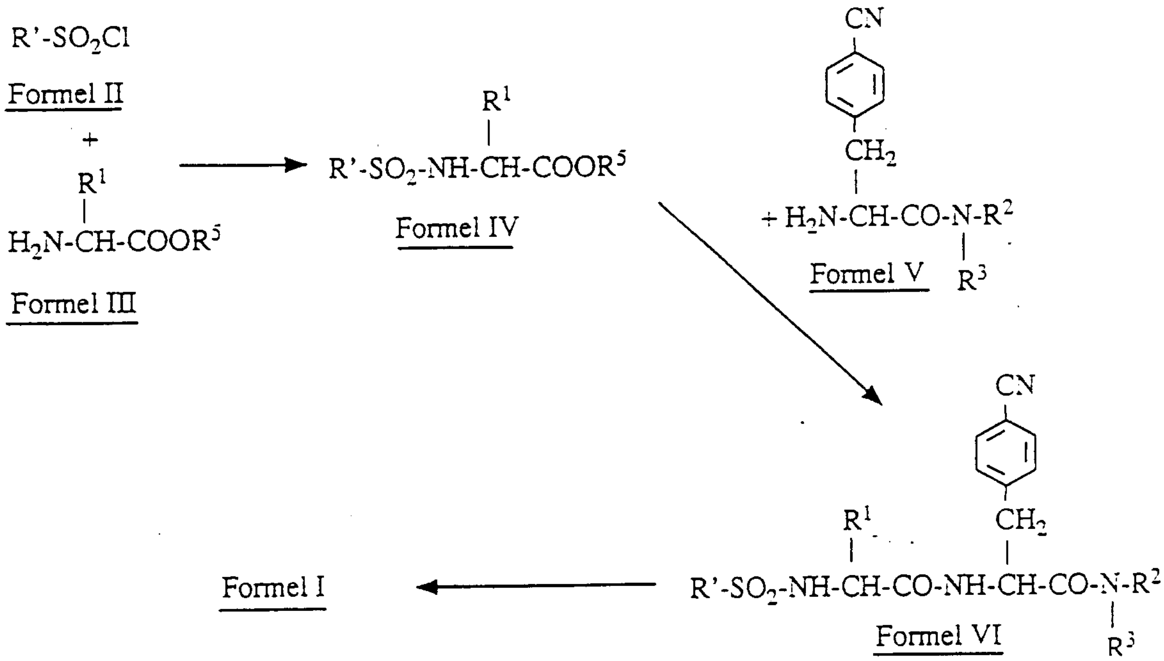

Formel II, Formel III, Formel IV, Formel V, Formel VI, Formel I

worin

R' ein in der alpha- oder beta-Position gebundener Naphthalinring ist, der gegebenenfalls mit Alkylgruppen, die bis zu 3 C-Atome enthalten, und/oder Alkoxygruppen mit jeweils bis zu 3 C-Atomen, derivatisiert ist, oder ein in der alpha- oder beta-Position gebundener Tetralinring oder Indanring ist, der gegebenenfalls mit Alkylgruppen, die aus bis zu 3 C-Atomen bestehen, und/oder auch Alkoxygruppen mit jeweils bis zu 3 C-Atomen derivatisiert ist, oder ein Phenylring ist, der gegebenenfalls mit Alkylgruppen, die bis zu 4 C-Atome enthalten, und/oder mit bis zu drei Gruppen der Struktur O-X, in der O Sauerstoff und X Wasserstoff, Methyl, Ethyl, n-Propyl, i-Propyl oder tert.-Butyl ist, und/oder mit einer Gruppe der Struktur -COOY, in der Y Wasserstoff, Methyl, Ethyl, n-Propyl, i-Propyl, tert.-Butyl, i-Butyl, i-Pentyl oder neo-Pentyl ist, derivatisiert ist, oder ein Chromansystem ist, das vorzugsweise mit bis zu 5 Alkylgruppen, die bis zu 3 C-Atome enthalten, derivatisiert ist, bedeutet

mit einer alpha-Amino-Verbindung der Formel III, worin

$R^1$ eine Gruppe der Struktur A-B ist, wobei A = $-(CH_2)_n$ und n = 1-4 und B eine Säurefunktion ist, ausgewählt aus der Gruppe Carboxylfunktion, die gegebenenfalls verestert sein kann oder als Amid vorliegt, wobei die Ester einen Alkohol mit bis zu 17 C-Atomen enthalten, Sulfonsäurefunktion, eine Funktion einer Säure des Phosphors, eine Boronsäurefunktion und Tetrazolgruppen, oder $R^1$ eine Gruppe der Struktur A-B-C ist, wobei A obige Bedeutung hat, B Carbonyl oder Sulfonyl ist und die Gruppe C sich von einer N-gebundenen alpha, beta, gamma oder delta Aminosäure oder der Gruppe der N-glycosidisch verknüpften Uronsäuren ableitet, und

$R^5$ H,$(C_1\text{-}C_4)$-Alkyl-, Allyl-, Benzyl- oder p-Methoxybenzylgruppe,

wobei falls erforderlich die reaktiven Hydroxy-, Amino- oder Carbonylgruppen mit in der Peptidchemie üblichen Schutzgruppen geschützt sind, bedeuten, in der Gegenwart einer Alkalilauge, eines Alkalicarbonates oder einer organischen Base in Wasser oder einem polaren Lösungsmittel, bevorzugt $(C_1\text{-}C_4)$-Alkohol, DMF oder Acetonitril zu einer Verbindung der Formel IV, wobei die Reste ihre vorher genannte Bedeutung beibehalten, umsetzt, in dem Produkt selektiv den Rest $R^5$ hydrolytisch mit HCl/Eisessig oder Trifluoressigsäure/Dichlormethan oder hydrogenolytisch in Gegenwart von Palladium/Kohle in einem $(C_1\text{-}C_4)$-Alkohol, Essigsäure oder Ethylacetat als Lösungsmittel abgespaltet, und das entstandene Produkt, worin $R^5$ ein Wasserstoffatom ist, mit einem Phenylalaninderivat der Formel V, worin

$R^2$ und $R^3$ gleich oder verschieden sein können und Alkylgruppen mit bis zu 4 C-Atomen sind oder zusammen einen heterocyclischen Ring mit bis zu 8 Ringgliedern bilden, der mit einer Hydroxygruppe oder einer Hydroxyalkylgruppe mit bis zu 3 C-Atomen derivatisiert sein kann und diese Hydroxygruppe gegebenenfalls verestert vorliegt, wobei die entsprechenden Säuren Carbonsäuren sind, die bis zu 17 C-Atome enthalten, und in der das mit * gezeichnete C-Atom in der R oder S Struktur, vorzugsweise aber in der R-Struktur vorliegt,

bedeuten, nach einem in der Peptidchemie üblichen Kondensationsverfahren zu einer geschützten Verbindung der Formel VI, wobei die Reste ihrer vorhergenannte Bedeutung beibehalten, umgesetzt, dann in dem erhaltenen Produkt die am Phenylalanylrest gebundene Cyanogruppe nach einem bekannten dreistufigen Verfahren

a) Umsetzung mit Schwefelwasserstoff/Triethylamin zu Thioamid
b) Umsetzung des Thioamids mit Methyliodid in Aceton zu Methylthiomid und
c) Umsetzung des Methylethioimides mit Ammoniumacetat in Methanol

zu einem Amidinoderivat der Formel I umsetzt und falls erforderlich, die Schutzgruppe nach in der Peptidchemie üblichen Verfahren abspaltet, wobei eine weitere Verbindung der Formel I entsteht, und anschließend die Verbindung der Formel I in ein anderes pharmakologisch verträgliches Salz überführt.

Die erfindungsgemäßen Inhibitoren werden zur Beurteilung ihrer Wirksamkeit nach verschiedenen Kriterien geprüft, vorzugsweise sind dies die Bestimmung des Ki-Wertes, des $IC_{50}$-Wertes und der partiellen Thromboplastinzeit (PTT) in vivo und in vitro. Zur Spezifitätsprüfung werden die $IC_{50}$-Werte gegen diverse Serinproteasen, insbesondere Thrombin und Trypsin, ermittelt. Die Stabilität der erfindungsgemäßen Substanzen wird bestimmt, indem eine Substanzprobe mit einem Reinenzym, bevorzugt Trypsin, Chymotrypsin oder Papain oder mit Leber- oder Darmhomogenaten inkubiert wird und aus den Lösungen in zeitlichen Abständen Proben entnommen werden, die vorzugsweise mit HPLC gemessen werden. Damit läßt sich zeigen, daß die beanspruchten Verbindungen gegenüber dem Stand der Technik stabiler sind und weniger rasch abgebaut werden. Bei den beanspruchten Verbindungen handelt es sich um spezifische und hochaktive Thrombininhibitoren mit einem beträchtlichen antithrombotischen Potential, das die bisher bekannten niedermolekularen Inhibitoren übertrifft, und die sich teilweise auch durch eine enterale Applikationsmöglichkeit auszeichnen, wie mit der Verbindung Mtr-L-Asp-D-Aph-Pip gezeigt werden konnte.

Gegenstand der Erfindung ist auch ein diagnostisches oder therapeutisches Mittel enthaltend eine Verbindung der Formel I.

Gegenstand der Erfindung ist außerdem die Verwendung einer Verbindung der Formel I in einem Verfahren zur Herstellung eines Diagnostikums oder eines Arzneimittels mit antithrombotischer Wirkung.

**Abkürzungen:**

| | |
|---|---|
| Aph | Amidinophenylalanin |
| NAPAP | β-Naphthylsulfonyl-glycyl-D,L-p-amidinophenylalanyl-piperidid |
| Asp | Asparaginsäure |
| Asn | Asparagin |
| Glu | Glutaminsäure |
| $Cys(SO_3H)$ | Cysteinsulfonsäure |
| β-Dmn | 6,7-Dimethoxynaphthyl |
| β-Mns | 5-Methoxynaphthyl |
| Pmc | 2,2,5,7,8,-Pentamethylchroman |
| Thn | 5,6,7,8,-Tetrahydronaphthalin |
| Tmn | 5,6,7,8,-Tetramethylnaphthyl |
| Phl | Phenyl- |
| Mtr | 4-Methoxy-2,3,6-trimethylphenyl- |
| Tos | 4-Methylphenyl |
| Pme | 2,3,4,5,6-Pentamethylphenyl- |
| Mte | 4-Methoxy-2,3,5,6-tetramethylphenyl- |
| Htr | 4-Hydroxy-2,3,6-trimethylphenyl- |
| Cph | 4-Carboxyphenyl- |
| Z (Cbo) | Benzyloxycarbonyl- |
| Boc | tert.-Butyloxycarbonyl |
| Bpoc | Biphenylisopropyloxycarbonyl- |
| Ddz | Dimethoxydimethylbenzyloxycarbonyl |
| Fmoc | Fluorenylmethyloxycarbonyl |
| Pip | Piperidin |
| OtBu | tert.-Butylester |
| OMe | Methylester |
| OEt | Ethylester |
| OiBu | iso-Butylester (sec-Butylester) |
| OiPr | iso-Propylester |

| | |
|---|---|
| OnPe | neo-Pentylester |
| DC | Dünnschichtchromatographie |
| DCCI | Dicyclohexylcarbodiimid |
| DMF | Dimethylformamid |
| FAB-MS | Fast atom bombardment mass spectrometry |
| DIPEA | Diisopropylethylamin |
| HOBt | Hydroxybenzotriazol |

Die folgenden Beispiele beschreiben die Erfindung näher:

**Beta-Naphthylsulfonyl-L-asparaginsäure-D-p-amidinopheny-l-alanyl-piperidid**

**1. Boc-D-p-cyanophenylalanyl-piperidid**

50 g (255 mMol) p-Cyanobenzylbromid, 55 g (255mMol) Acetamidomalonsäurediethylester und 2g Kaliumjodid wurden in 250 ml abs. Dioxan zum Sieden erhitzt. Hierzu wurde innerhalb von 3 Stunden eine frisch hergestellte Lösung von 6g (260 mMol)Natrium in Ethanol zugetropft. Nach weiteren 3 Stunden Rückflußkochen wurde auf 80 Grad abgekühlt und innerhalb von 3 Stunden 170 ml 3N Natronlauge zugesetzt. Der Ansatz wurde 4 Stunden auf 95 Grad erhitzt. Nach Abkühlung wurde mit 6N HCl auf pH 1 eingestellt und das Dioxan abgedampft. Ein eventuell ausgefallener Niederschlag wurde abfiltriert. Es wurde mit Natronlauge auf pH 9 eingestellt und 2 mal mit Ethylacetat extrahiert. Die wässrige Phase wurde nochmals mit Salzsäure auf pH 1 eingestellt, wobei N-Acetyl-cyanophenylalanin auskristallisierte. Die Kristalle wurde gesammelt, mehrmals mit Wasser gewaschen und im Hochvakuum getrocknet.
Ausbeute: 47 g (79,2% d.Th.)
Reinheitsprüfung: DC Rf = 0.5 (Chloroform 50/Methanol 10/Eisessig 2.5//Volumenanteile)
24 g dieses Produktes wurden durch Zugabe von 3 N Natronlauge in 3 Liter Wasser gelöst und der pH wurde auf 6-6,5 eingestellt. Dazu wurden 500 mg Acylase zugesetzt und der Ansatz wurde 4 Tage bei 37 Grad inkubiert. Hierauf wurde die Lösung durch Ultrafiltration von der Acylase befreit und anschließend auf ein Volumen von 1 Liter eingeengt. Nach Einstellung auf pH 1 wurde mehrfach mit Ethylacetat extrahiert. Die organische Phase wurde mit wenig konz. Kochsalzlösung gewaschen, über Natriumsulfat getrocknet und das Lösemittel eingedampft. Man erhielt 8.2 g N-Acetyl-D-cyanophenylalanin (68% d.Th.). Zu 8 g dieser Verbindung wurden 22 ml Eisessig und 4,3 ml konz. Salzsäure mit 40 ml Wasser gegeben und 24 Stunden zum Sieden erhitzt. Nach Abdampfen der Abspaltlösung und Nachschleppen anhaftender Säurespuren mit Methanol wurde aus Methanol/Diethylether umgefällt.
Ausbeute: 6.6g (85% d.Th.)
5 g D-Cyanophenylalaninhydrochlorid wurden in 14 ml Wasser unter Zugabe von 7.5 ml Diisopropylethylamin gelöst. Dazu wurde eine Lösung von 6 g tert.-Butyloxycarbonyl-oxyimino-2-phenylacetonitril in 17 ml Dioxan zugesetzt und über Nacht gerührt. Es wurden 40 ml Wasser und 50 ml Ethylacetat zugesetzt. Die Wasserphase wurde abgetrennt und die organische Phase nochmals mit 1M Kaliumhydrogencarbonat extrahiert. Die vereinigten wässrigen Phasen wurden nochmals mit 10 ml Diethylether gewaschen und anschließend mit Salzsäure auf pH 3 eingestellt. Es wurde 3 mal mit Ethylacetat extrahiert, mit Natriumchloridlösung gewaschen und die organische Phase wurde über Natriumsulfat getrocknet. Nach Abdampfen des Lösemittels erhielt man 5.6 g (78%) Boc-D-cyanophenylalanin. 3.26 g (10 mMol) Boc-D-cyanophenylalanin, 1.49 g(11 mMol) HOBt und 2.42 g(12 mMol) DCCI wurden in 50 ml DMF gelöst und 1 Stunde gerührt. Es wurde 1 ml Piperidin zugesetzt und über Nacht gerührt. Ausgefallener Dicyclohexylharnstoff wurde abfiltriert, das DMF abdestilliert und der Rückstand in Ethylacetat aufgenommen. Es wurde 3 mal mit Kaliumhydrogencarbonat, 3 mal mit 1M Kaliumhydrogensulfat und 3 mal mit ges. Kochsalzlösung gewaschen. Nach Trocknung der organischen Phase mit Natriumsulfat und Abdestillation des Lösemittels erhielt man 3.16 g (80%) Boc-D-Cyanophenylalanyl-piperidid
Reinheitskontrolle: DC Rf = 0.27 (Chloroform)

**2. D-cyanophenylalanyl-piperidin-hydrochlorid**

3 g der Boc-geschützten Verbindung wurden in 50 ml 1.2 N HCl in Eisessig gelöst und 30 min bei Raumtemperatur gerührt. Das Abspaltreagens wurde im Vakuum abdestilliert, mit Toluol nachgeschleppt und der Rückstand mit wenig Diethylether verrieben. Die Kristalle wurden gesammelt und im Vakuum getrocknet.
Ausbeute: 2.2 g (90 % d.Th.)

**3. Ddz-Asp(tBu)-D-cyanophenylalanyl-piperidid**

2.88 g (7 mMol) Ddz-Asp(tBu), 1.04 g HOBt, 1.73g DCCI und 2.06g (7 mMol) Cyano-phenylalanyl-piperidid Hydrochlorid wurden in 50 ml DMF gelöst. Nach Zusatz von 2.4ml (14 mMol) Diisopropylethylamin wurde 1 Tag im Dunklen bei Raumtemperatur gerührt. Das Lösemittel wurde im Vakuum abdestilliert, der Rückstand in Ethylacetat aufgenommen und 3 mal mit 1M Kaliumhydrogensulfatlösung, 3 mal mit Kaliumhydrogencarbonatlösung und 2 mal mit konz. Kochsalzlösung gewaschen. Die organische Phase wurde über Natriumsulfat getrocknet und das Lösemittel abgedampft. Der Rückstand wurde mit Diisopropylether verrührt, die Kristalle gesammelt und getrocknet. Man erhielt 3.86 g (85% d.Th.) Ddz-Asp(tBu)-D-cyano-phenylalanyl-piperidid.

**4. β-Naphthylsulfonyl-Asp(tBu)-D-cyanophenylalanyl-piperidid**

3.25 g (5 mMol) Ddz-Asp(tBu)-D-cyanophenylalanylpiperidid wurden in 200 ml 5 % iger Trifluoressigsäure in Dichlormethan gelöst und 30 min bei Raumtemperatur gerührt. Der Ansatz wurde auf 2 M Natriumhydrogencarbonatlösung gegossen, die organische Phase zweimal mit Natriumhydrogencarbonatlösung und einmal mit konz. Kochsalzlösung gewaschen. Nach Trocknung über Natriumsulfat wurde das Lösemittel abgedampft und der ölige Rückstand wurde dreimal mit Diisopropylether extrahiert. Der ölige Rückstand wurde unter Zugabe von 1.71 ml (10 mmol) DIPEA in Dichlormethan gelöst, wozu unter Rühren 1.13 g (5 mmol) β-Naphthylsulfonylchlorid gegeben wurde. Der Ansatz wurde über Nacht bei Raumtemperatur gerührt und anschließend wurde die organische Phase je 3 mal mit $NaHCO_3$-Lösung, Kaliumhydrogensulfatlösung und Natriumchloridlösung gewaschen. Nach Trocknung der organischen Phase über Natriumsulfat wurde das Lösemittel abdestilliert und die erhaltene Substanz wurde ohne Reinigung zur weiteren Umsetzung benutzt.

**5. β-Naphthylsulfonyl-Asp(tBu)-D-amidino-phenylalanyl-piperidid**

Die in 4. erhaltene Verbindung wurden in 30 ml trockenem Pyridin gelöst und nach Zugabe von 1 ml Triethylamin wurde 3 Stunden lang Schwefelwasserstoffgas eingeleitet. Nach dreitägigem Stehenlassen bei Raumtemperatur wurde auf eine Mischung aus 100 g Eis und 50 ml konz. Salzsäure gegossen. Der Niederschlag wurde abgesaugt und mit Wasser gewaschen. Das Thioamid wurde nach Trocknung in 50 ml Aceton aufgenommen und mit 1.5 ml Methyljodid versetzt. Es wurde 30 Minuten am Rückfluß gekocht. Nach Abkühlen wurde mit Diethylether gefällt. Der Niederschlag wurde in Dichlormethan gelöst und zweimal mit Wasser gewaschen. Nach Trocknung der organischen Phase über Natriumsulfat und Entfernung des Lösemittels wurde der Rückstand in 30 ml trockenem Methanol aufgenommen und 200 mg Ammoniumacetat zugegeben. Es wurde 3 Stunden auf 60 Grad erhitzt. Das Lösemittel wurde im Vakuum abgedampft . Das Produkt wurde einer chromatographischen Reinigung an Sephadex LH-20 in Methanol unterzogen.
Ausbeute: 630 mg
Reinheitskontrolle: DC: RF = 0.55 (Chloroform 50/Methanol 10/ Eisessig 2.5 //Volumina)
FAB-MS: M + H 636

**6. β-Naphthylsulfonyl-Asp-D-amidino-phenylalanylpiperidid**

500 mg der unter 5. isolierten Substanz wurden in 5 ml 1.2 N HCl in Eisessig gelöst und 1 Stunde bei Raumtemperatur gerührt. Nach Abdampfen des Lösemittels wurde der Rückstand mit Diethylether verrieben und auf einer Filterfritte gesammelt. Das Produkt wurde über Phosphorpentoxid im Hochvakuum getrocknet. Die Rohsubstanz wurde in Wasser gelöst und an CM-Fraktogel Ionenaustauscher gebunden. Die Elution erfolgte mit 5%-iger Essigsäure. Nach Lyophilisation erhielt man 290 mg des Acetatsalzes. Die korrekte Struktur wurde mittels $^{13}C$-NMR-Spektroskopie bestätigt.
FAB-MS: M + H 580

**6,7-Dimethoxy-β-naphthalinsulfonyl-L-Asp-D-p-amidino-phenylalanylpiperidid**

Die Herstellung erfolgte in Analogie zu dem obigen Beispiel. Anstelle von β-Naphthalinsulfonsäurechlorid wurde 6,7-Dimethoxy-β-naphthalinsulfonsäurechlorid eingesetzt.
FAB-MS: M + H 640

**5,6,7,8-Tetrahydro-β-naphthalinsulfonyl-L-Asp-D-p-amidinophenylalanylpiperidid**

Unter Verwendung von 5,6,7,8-Tetrahydro-β-naphthalinsulfonyl-chlorid und des obigen Verfahrens wurde 5,6,7,8-Tetrahydro-β-naphthalinsulfonyl-L-Asp-D-p-amidinophenylalanylpiperidid hergestellt.
FAB-MS: M + H 584

**4-Methoxy-2,3,6-Trimethylphenylsulfonyl-L-Asp-D-p-amidinophenylalanylpiperidid**

Unter Verwendung von 4-Methoxy-2,3,6-trimethylphenylsulfonyl-chlorid und des obigen Verfahrens wurde 4-Methoxy-2,3,6-trimethylphenylsulfonyl-L-Asp-D-p-amidinophenylalanylpiperidid hergestellt.
FAB-MS: M + H 602

**4-Methoxy-2,3,6-trimethylphenylsulfonyl-L-Cys($SO_3H$)-D-p-amidinophenylalanylpiperidid**

Unter Verwendung von 4-Methoxy-2,3,6-trimethyl-phenylsulfonyl-chlorid und des obigen Verfahrens wurde 4-Methoxy-2,3,6-trimethylphenylsulfonyl-L-Cys($SO_3H$)-D-p-amidinophenylalanylpiperidid hergestellt.
FAB-MS: M + H 638

**4-Methoxy-2,3,6-trimethylphenylsulfonyl-L-asparaginyl-N-[β-D-glucopyranosyl)uronsäure]-D-p-amidinophenyl-alanylpiperidid (Mtr-L-Asp(β-D-Aminoglucuronsäure)-D-Aph-pip)**

**1. Methyl-(2,3,4-tri-O-acetyl-β-D-glucopyranosylamin)uronat**

2 g Methyl-(2,3,4-tri-O-acetyl-β-D-glucopyranosyl-azid)uronat wurden in 200 ml Ethylacetat/Methanol (2:1;V:V) gelöst, 2g Palladium/Kohle zugesetzt, mit Triethylamin auf pH 7.5 gestellt und am Durchfluß eine Stunde mit Wasserstoff behandelt. Die Reaktionsmischung wurde abfiltriert und das Lösemittel abgedampft.
Ausbeute: 1.9 g
Reinheitskontrolle: DC Rf = 0.35 (Dichlormethan:Aceton / 2:1)

**2. 2-N-(Z)-4-N-[Methyl-(2,3,4-tri-O-acetyl-β-D-glucopyranosyl)uronat]-L-asparagin-tert.butylester**

1.26 g Z-Asparaginsäure-alpha-tert.-butylester, 0.9g HOBt und 1.4 g Dicyclohexylcarbodiimid wurden in 100 ml THF gelöst und bei O° C mit 1.4 g der Stufe 1 versetzt. Es wurde über Nacht bei Raumtemperatur gerührt und das Lösemittel im Vakuum eingedampft. Der Rückstand wurde in Chloroform aufgenommen, mit Wasser gewaschen, mit Natriumsulfat getrocknet und das Lösemittel abdestilliert. Der Rückstand wurde an Kieselgel mittels Chloroform/Aceton (6:1/V:V) gereinigt.
Ausbeute: 1.7 g
Reinheitskontrolle: DC Rf = 0.72 (Chloroform:Aceton/6:1)

**3. 4-N-[Methyl-(2,3,4-tri-O-acetyl-β-D-glucopyranosyl) uronat]-L-asparagin-tert.butylester**

0.9 g der 2. Stufe wurden in 20 ml Methanol gelöst, mit einer Spatelspitze Palladium/Kohle versetzt und 5 Stunden hydriert. Nach Abfiltrieren des Katalysators wurde das Lösemittel abdestilliert und der Rückstand (0.75 g) ohne weitere Reinigung zur nächsten Reaktion eingesetzt.

**4. 2-N-(4-Methoxy-2,3,6-trimethyl-benzolsulfonyl)-4-N-[methyl-(2,3,4-tri-O-acetyl-β-D-glucopyranosyl-)uronat]-L-asparagin-tert.butylester**

1.73 g der vorhergehenden Stufe, 1.2 ml Diisopropylethylamin und 0.8 g Mtr-chlorid wurden in 80 ml DMF gelöst und über Nacht bei Raumtemperatur gerührt. Das Lösemittel wurde im Vakuum abgedampft, der Rückstand in Ethylacetat aufgenommen und mit Wasser dreimal ausgewaschen. Die organische Phase wurde mit Natriumsulfat getrocknet und im Vakuum abgedampft. Zur weiteren Reinigung wurde an Kieselgel mit Dichlormethan/Aceton (3:1/V:V) chromatographiert.
Ausbeute: 1.57 g
Reinheitskontrolle: DC Rf = 0.87 (Dichlormethan:Methanol/10:1)

**5. 2-N-(4-Methoxy-2,3,6-trimethyl-benzolsulfonyl)-4-N-[methyl-(2,3,4-tri-O-acetyl-β-D-glucopyranosyl-)uronat]-L-asparagin**

2.2 g aus der 4. Stufe wurden in 50 ml Trifluoressigsäure/Dichlormethan (1:1/V:V) gelöst und 1 Stunde bei Raumtemperatur gerührt. Das Säuregemisch wurde im Vakuum abdestilliert und anhaftende Säurespuren mittels Toluol im Vakuum abgedampft. Das erhaltene Produkt wurde ohne weitere Reinigung zur nächsten Umsetzung gebracht.
Ausbeute: 1.2 g
Reinheitskontrolle: DC Rf = 0.7 (Chloroform:Methanol/3:1)

**6. 2-N-(4-Methoxy-2,3,6-trimethyl-benzolsulfonyl)-4-N-[methyl-(2,3,4-tri-O-acetyl-β-D-glucopyranosyl-)uronat]-L-asparaginyl-D-4-amidinophenylalanin-piperidid**

1.0 g aus der vorhergehenden Stufe, 0.23 g HOBt und 0.37 g DCCI wurden in 50 ml DMF gelöst und bei 4° C 30 Minuten gerührt. Daraufhin wurden 0.4 g D-4-Amidinophenylalanin-piperidid und 0.5 ml N-Methyl-morpholin zugesetzt. Der Ansatz wurde über Nacht bei Raumtemperatur gerührt, abfiltriert und das Lösemittel im Vakuum abgedampft. Das Rohprodukt wurde an Kieselgel mit Chloroform/Methanol 5:1 chromatographiert.
Ausbeute: 1.2 g
Reinheitskontrolle: DC Rf = 0.7 (Chloroform:Methanol/3:1)

**7. 2-N-(4-Methoxy-2,3,6-trimethyl-benzolsulfonyl)-4-N-[methyl-(β-D-glucopyranosyl)uronat]-L-asparaginyl-D-4-amidinophenylalanin-piperidid**

1.1 g der vorhergehenden Stufe wurden in 50 ml Methanol gelöst und mit 1N Natronlauge auf pH 9 gestellt. Nach 2 Stunden bei pH 8.5-9 wurde mit methanolischer HCl neutralisiert und das Lösemittel im Vakuum abgedampft. Der Rückstand wurde in Methanol an Sephadex$^R$ LH-20 chromatographiert.
Ausbeute: 0.9 g
Reinheitskontrolle: DC Rf = 0.34 (Chloroform:Methanol:Wasser/40:20:1)

**8. 2-N-(4-Methoxy-2,3,6-trimethyl-benzolsufonyl)-4-N-(β-D-glucopyranosyl)uronat-L-asparaginyl-D-4-amidinophenylalanin-piperidid**

0.9 g der letzten Stufe wurden in 100 ml Chloroform: Methanol:Wasser (40:20:1/V:V:V) aufgenommen und mit 0.2 g Bariumhydroxid versetzt. Die Mischung wurde 3 Stunden bei Raumtemperatur gerührt, mit HCl in Methanol auf pH 3.5 gestellt und das Lösemittel abgedampft. Der Rückstand wurde in Methanol an Sephadex$^R$ LH-20 chromatographiert.
Ausbeute: 0.72 g
Reinheitskontrolle: DC Rf = 0.32 (Chloroform:Methanol:Wasser/8:6:1)
FAB-MS: 777

**4-Methoxy-2,3,6-trimethylbenzolsulfonyl-L-asparaginsäure-(gamma-aminobuttersäure)-D-Aph-piperidid**

**1. 4-Methoxy-2,3,6-trimethylbenzolsulfonyl-L-asparaginsäure-(gamma-aminobuttersäure-tert.-butylester)-D-Cyanophenylalanin-piperidid**

1 g Mtr-Asp-D-Cyanophenylalanin-piperidid wurden in 30 ml DMF gelöst und mit 0.34 ml Diisopropylethylamin, 0.39g HOBt, 0.42g Gamma-Aminobuttersäure-tert.-butylester und 0.66g Dicyclohexylcarbodiimid versetzt. Es wurde zunächst eine Stunde im Eisbad, dann über Nacht bei Raumtemperatur gerührt. Der ausgefallene Dicyclohexylharnstoff wurde abfiltriert und das Lösemittel im Vakuum abgedampft. Der ölige Rückstand wurde in Ethylacetat aufgenommen und je 2 mal mit Wasser, 1M Kaliumhydrogencarbonatlösung, 1M Kaliumhydrogensulfatlösung und gesättigter Kochsalzlösung gewaschen. Nach Trocknung über Natriumsulfat wurde das Lösemittel abdestilliert.
Ausbeute: 1.1 g
Reinheitskontrolle: DC Rf = 0.89 (Chloroform:Methanol/9:1)

**2. 4-Methoxy-2,3,6-trimethylbenzolsulfonyl-L-asparaginsäure-(gamma-aminobuttersäure-tert.-butylester)-D-Aph-piperidid**

Die Überführung der Cyanogruppe in die Amidinogruppe erfolgte wie im 1. Beispiel 5. Stufe.
Ausbeute: 500 mg
Reinheitskontrolle: DC Rf = 0.4 (Chloroform:Methanol:Essigsäure/50:10:2.5)

**3. 4-Methoxy-2,3,6-trimethylbenzolsulfonyl-L-asparaginsäure-(gamma-aminobuttersäure)-D-Aph-piperidid**

400 mg der vorhergehenden Stufe wurden in 15 ml 1.2 N HCl/Essigsäure aufgenommen und 90 Minuten bei Raumtemperatur gerührt. Die Säuremischung wurde abdestilliert und zweimal mit Toluol vermischt und das Toluol abgedampft. Das Rohprodukt wurde in 3 ml Methanol gelöst und durch Eintropfen in 50 ml Diethylether kristallisiert. Der Niederschlag wurde gesammelt und im Vakuum getrocknet.
Ausbeute: 320 mg
Reinheitskontrolle: DC Rf = 0.25 (Chloroform:Methanol:Essigsäure/50:10:2.5)
FAB-MS: 686
Unter Auslassung der Razematspaltung mittels Acylase im Schritt 1. wurden die entsprechenden racemischen D,L-Cyanophenylalanin-piperidideebenfalls zu den erfindungsgemäßen Verbindungen umgesetzt, die ebenfalls hochpotente Thrombinhemmstoffe darstellen.

**Tabelle I Zusammenfassung von erfindungsgemäßen Verbindungen***

ß-Naphthylsulfonyl-L-Glu-D-Aph-Pip
ß-Naphthylsulfonyl-L-Asp-D-Aph-Pip
ß-Naphthylsulfonyl-D-Asp-D,L-Aph-Pip
ß-Naphthylsulfonyl-L-Asp-D,L-Aph-Pip
ß-Naphthylsulfonyl-L-Asp-D,L-Aph-3-Hydroxymethylpiperidid
ß-Naphthylsulfonyl-L-Asp(OtBu)-D-Aph-Pip
ß-Naphthylsulfonyl-L-Asp(OMe)-D-Aph-Pip
ß-Naphthylsulfonyl-L-Asp(OEt)-D-Aph-Pip
ß-Naphthylsulfonyl-L-Asp(OiBu)-D-Aph-Pip
Mtr-D-Asp-D,L-Aph-Pip
Mtr-L-Asp(OtBu)-D-Aph-Pip
Mtr-L-Asp(OMe)-D-Aph-Pip
Mtr-L-Asp(OEt)-D-Aph-Pip
Mtr-L-Asp(OiBu)-D-Aph-Pip
Mtr-L-Asp(OiPr)-D-Aph-Pip
Mtr-L-Asp-D-Aph-Pip
Mte-L-Asp-D-Aph-Pip
Mte-L-Asp(OiBu)-D,L-Aph-Pip
Htr-L-Asp(OtBu)-D-Aph-Pip
Htr-L-Asp-D-Aph-Pip
Mtr-L-Glu-D,L-Aph-Pip
Mtr-L-Asn-D-Aph-Pip
Mtr-D-Asn-D,L-Aph-Pip
Thn-L-Asn-D-Aph-Pip
Pme-L-Asp(OtBu)-D-Aph-Pip
Pme-L-Asp-D-Aph-Pip
Pme-L-Asp(OtBu)-D,L-Aph-Pip
Phl-L-Asp(OtBu)-D-Aph-Pip
Phl-L-Asp-D-Aph-Pip
ß-Dmn-L-Asp(OtBu)-D-Aph-Pip
ß-Dmn-L-Asp-D-Aph-Pip
Tos-L-Asp(OtBu)-D-Aph-Pip

Tos-L-Asp-D-Aph-Pip
Pmc-L-Asp(OtBu)-D-Aph-Pip
Pmc-L-Asp-D-Aph-Pip
ß-Mns-L-Asp(OnPe)-D-Aph-Pip
ß-Mns-L-Asp-D-Aph-Pip
Cph-L-Asp(OtBu)-D,L-Aph-Pip
Cph-L-Asp-D,L-Aph-Pip
Phl-L-Cys($SO_3H$)-D-Aph-Pip
Mtr-L-Cys($SO_3H$)-D,L-Aph-Pip
Mtr-L-Cys($SO_3H$)-D-Aph-Pip
Mtr-L-Asp(gamma-Aminobuttersäure)-D-Aph-Pip
Mtr-L-Asp(L-Threonin)-D-Aph-Pip
Mtr-L-Asp(L-Phenylalanin)-Aph-Pip
Mtr-L-Asn[(ß-D-glucopyranosyl)-uronat]-D-Aph-Pip

* Die Salzformen an der Amidinofunktion sind nicht genannt

**Bestimmung der Inhibitionskonstanten für Thrombin**

Die Inhibitionskonstanten (Ki) für die Substanzen wurden enzymkinetisch nach bekannten Verfahren bestimmt. Das eingesetzte humane Thrombin wurde als 87% rein mit Hilfe der "active site titration" bestimmt. Die Testlösung für die Ki-Bestimmung bestand aus Puffer (50 mM Tris-HCl, 75 mM NaCl, pH7,8, 37 Grad C), 100 pM Thrombin, 0,1 mM Substrat S2238 (Kabi) und Inhibitor, der einen Bereich von 0 bis 0,2 µM umspannte. Inhibitor und Enzym wurden 10 Minuten vorinkubiert, die Reaktion wurde gestartet durch Zugabe des chromogenen Substrats S2238. Für die Auswertung der Kinetiken wurde der mathematische Algorithmus für "tight-binding" verwendet, der mit Hilfe der nicht-linearen Regression Ki-Werte (Tabelle II) und den Hemmtyp ergab. Der Hemmtyp für alle Inhibitoren wurde als größer 90 % kompetitiv ermittelt.

**Bestimmung der Spezifität der Inhibitoren**

Die Spezifität der Inhibitoren wurde gegenüber Thrombin und Trypsin bestimmt. Die Spezifität ist definiert als das Verhältnis der Ki-Werte für Trypsin und Thrombin (Tabelle II). Die Konzentration an Inhibitor die eine 50%-ige Hemmung der Enzymaktivität bewirkte, wurde als $IC_{50}$ bezeichnet (100% entspricht der nicht inhibierten Enzymreaktion). Der $IC_{50}$-Wert für Trypsin wurde folgendermaßen ermittelt: Trypsin aus Rinderpankreas wurde mit steigenden Konzentrationen Inhibitor in 25 mM Tris-HCl, 10 mM $CaCl_2$, pH 7,8 bei 37 Grad C 10 Min. vorinkubiert. Die enzymatische Reaktion wurde gestartet durch Zugabe des Substrats Chromozym TRY ($7{,}1 \times 10^{-5}$ M). Die Freisetzung von pNA wurde nach einer Stunde bei 405 nm gemessen. In analoger Weise wurden die IC50-Werte für Thrombin bestimmt, mit den Ausnahmen, daß humanes Thrombin, Puffer (50 mM Tris-HCl, 50 mM NaCl, pH 7,8) und $5 \times 10^{-5}$ M S 2238 verwendet wurden. Aus dem $IC_{50}$-Wert für Thrombin und Trypsin wurden die Ki-Werte berechnet (Ki = $IC_{50}$/S + KM). Das Verhältnis der Trypsin- und Thrombinwerte ergab die Spezifitäten. Die Ergebnisse sind in der Tabelle II zusammengefaßt.

Tabelle II

| Wirksamkeiten einiger ausgewählter Verbindungen | | |
|---|---|---|
| Verbindung | Spezifität Thrombin/Trypsin | Thrombinhemmung Ki(nanomol/l) |
| 1. Napap | 24 | 11 |
| 2. Nas-Asp-D-Aph-Pip | 7 | 10 |
| 3. Dmn-Asp-D-Aph-Pip | 230 | 6 |
| 4. Mtr-Asp-D-Aph-Pip | 125 | 1.5 |
| 5. Mtr-Asp(tBu)-Aph-Pip | 297 | 0.9 |
| 6. Mtr-Asp(iPr)-Aph-Pip | 378 | 0.6 |
| 7. Nas-Asn-Aph-Pip | 48 | 6 |
| 8. Mtr-Asn[(β-glucopyranosyl)uronat]-D-Aph-pip | 4106 | 0.085 |
| 9. Mtr-L-Cys($SO_3H$)-D-Aph-pip | 150 | 0.6 |
| 10. Mtr-l-Asp(gamma-aminobuttersäure)-D-Aph-pip | 1810 | 0.8 |

## Patentansprüche

**Patentansprüche für folgende Vertragsstaaten : AT, BE, CH, DE, DK, FR, GB, IT, LI, LU, NL, PT, SE**

**1.** Verbindung der Formel I

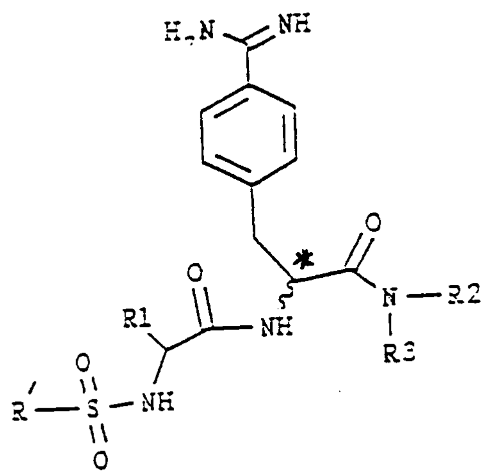

I

worin

R' ein in der alpha- oder beta-Position gebundener Naphthalinring ist, der gegebenenfalls mit Alkylgruppen, die bis zu 3 C-Atome enthalten, und/oder Alkoxygruppen mit jeweils bis zu 3 C-Atomen, derivatisiert ist, oder ein in der alpha- oder beta-Position gebundener Tetralinring oder Indanring ist, der gegebenenfalls mit Alkylgruppen, die aus bis zu 3 C-Atomen bestehen, und/oder auch Alkoxygruppen mit jeweils bis zu 3 C-Atomen derivatisiert ist,
oder ein Phenylring ist, der gegebenenfalls mit Alkylgruppen, die bis zu 4 C-Atome enthalten, und/oder mit bis zu drei Gruppen der Struktur O-X, in der O Sauerstoff und X Wasserstoff, Methyl, Ethyl, n-Propyl, i-Propyl oder tert.-Butyl ist, und/oder mit einer Gruppe der Struktur -COOY, in der Y Wasserstoff, Methyl, Ethyl, n-Propyl, i-Propyl, tert.-Butyl, i-Butyl, i-Pentyl oder neo-Pentyl ist, derivatisiert ist,
oder ein Chromansystem ist, das vorzugsweise mit bis zu 5 Alkylgruppen, die bis zu 3 C-Atome enthalten, derivatisiert ist,

$R_1$ eine Gruppe der Struktur A-B ist, wobei A = $-(CH_2)_n-$ und n = 1-4 und B eine Säurefunktion ist, ausgewählt aus der Gruppe Carboxylfunktion, die gegebenenfalls verestert sein kann oder als Amid vorliegt, wobei die Ester einen Alkohol mit bis zu 17

C-Atomen enthalten, Sulfonsäurefunktion, eine Funktion einer Säure des Phosphors, eine Boronsäurefunktion und Tetrazolgruppe, oder $R_1$ eine Gruppe der Struktur A-B-C ist, wobei A obige Bedeutung hat, B Carbonyl oder Sulfonyl ist und die Gruppe C sich von einer N-gebundenen alpha, beta, gamma oder delta Aminosäure oder der Gruppe der N-glycosidisch verknüpften Uronsäuren ableitet, und

$R_2$ und $R_3$ gleich oder verschieden sein können und Alkylgruppen mit bis zu 4 C-Atomen sind oder zusammen einen heterocyclischen Ring mit bis zu 8 Ringgliedern bilden, der mit einer Hydroxygruppe oder einer Hydroxyalkylgruppe mit bis zu 3 C-Atomen derivatisiert sein kann und diese Hydroxygruppe gegebenenfalls verestert vorliegt, wobei die entsprechenden Säuren Carbonsäuren sind, die bis zu 17 C-Atome enthalten, und in der das mit * gezeichnete C-Atom in der R oder S Struktur, vorzugsweise aber in der R-Struktur vorliegt.

**2.** Verbindung nach Anspruch 1, dadurch gekennzeichnet, daß das mit * gekennzeichnete C-Atom in der R-Struktur vorliegt.

**3.** Eine Verbindung nach Anspruch 1, in der R' $\beta$-Naphthyl, $R_1$ $-CH_2-COOX$ mit X gleich Wasserstoff und $R_2$ und $R_3$ zusammen Piperidin sind.

**4.** Eine Verbindung nach Anspruch 1, in der R' $\beta$-6,7-Dimethoxynaphthyl-,$R_1$ $-CH_2-COOX$ mit X gleich Wasserstoff und $R_2$ und $R_3$ zusammen Piperidin sind.

**5.** Eine Verbindung nach Anspruch 1, in der R' $\beta$-Tetralin, $R_1$ $-CH_2-COOX$ mit X gleich Wasserstoff und $R_2$ und $R_3$ zusammen Piperidin sind.

**6.** Eine Verbindung nach Anspruch 1, in der R' 4-Methoxy-2,3,6-trimethylphenyl-, $R_1$ $-CH_2-COOX$ mit X gleich Wasserstoff und $R_2$ und $R_3$ zusammen Piperidin sind.

**7.** Eine Verbindung nach Anspruch 1, in der R' 4-Carboxyphenyl-, $R_1$ $-CH_2-COOX$ mit X gleich Wasserstoff und $R_2$ und $R_3$ zusammen Piperidin sind.

**8.** Eine Verbindung nach Anspruch 1, in der R' 4-Hydroxy-2,3,6,-trimethylphenyl-,$R_1$ $-CH_2-COOX$ mit X gleich Wasserstoff und $R_2$ und $R_3$ zusammen Piperidin sind.

**9.** Eine Verbindung nach Anspruch 1, in der R' 2,2,5,7,8,-Pentamethylchroman-, $R_1$ $-CH_2-COOX$ mit X gleich Wasserstoff und $R_2$ und $R_3$ zusammen Piperidin sind.

**10.** Eine Verbindung nach einem der Ansprüche 1-8, dadurch gekennzeichnet, daß X einen Alkoholrest darstellt, der bis zu 17 C-Atome aufweist.

**11.** Eine Verbindung nach einem der Ansprüche 1-8, wobei $R_1$ die Struktur $-(CH_2)_n-SO_3H$ mit $n = 1$ bis 4 aufweist.

**12.** Eine Verbindung nach einem der Ansprüche 1-8, wobei R1 die Struktur $-(CH_2)_n-PO(OH)_2$ mit $n = 1$ bis 4 aufweist.

**13.** Eine Verbindung nach einem der Ansprüche 1-10, dadurch gekennzeichnet, daß $R_2$ und $R_3$ zusammen 3-Hydroxymethylpiperidin sind.

**14.** Verbindung nach Anspruch 13, dadurch gekennzeichnet, daß die Hydroxyfunktion mit einer Carbonsäure verestert ist, die bis zu 17 C-Atome enthält.

**15.** Verfahren zur Herstellung einer Verbindung der Formel I in Anspruch 1, dadurch gekennzeichnet, daß man eine Verbindung der Formel II

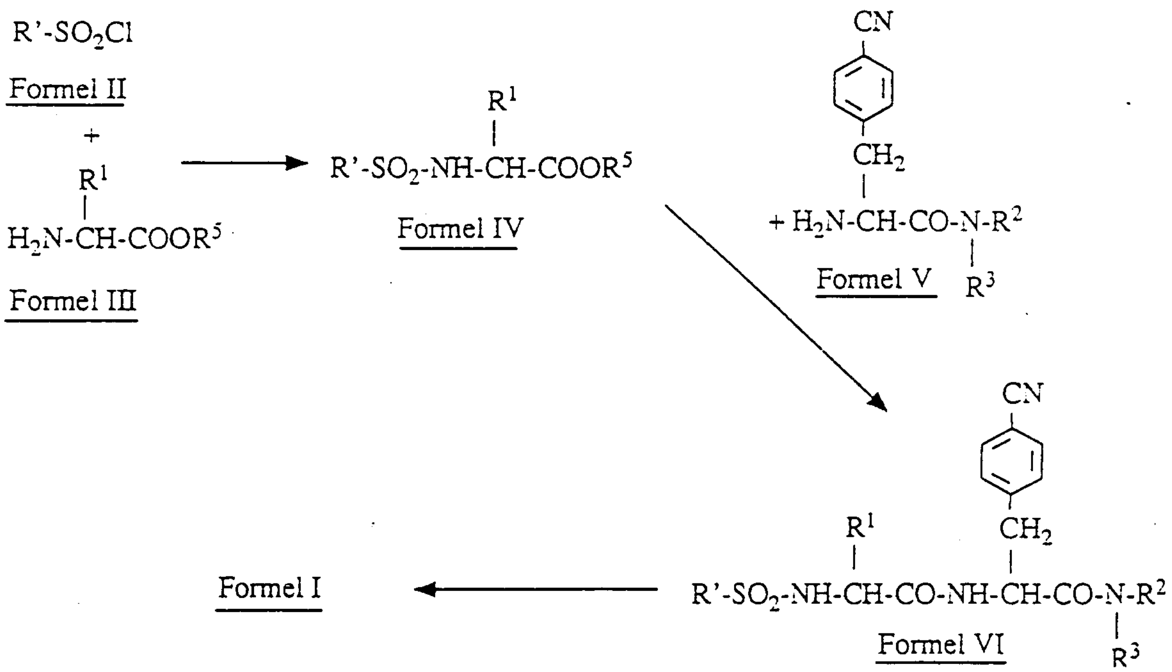

worin

R' ein in der alpha- oder beta-Position gebundener Naphthalinring ist, der gegebenenfalls mit Alkylgruppen, die bis zu 3 C-Atome enthalten, und/oder Alkoxygruppen mit jeweils bis zu 3 C-Atomen, derivatisiert ist, oder ein in der alpha- oder beta-Position gebundener Tetralinring oder Indanring ist, der gegebenenfalls mit Alkylgruppen, die aus bis zu 3 C-Atomen bestehen, und/oder auch Alkoxygruppen mit jeweils bis zu 3 C-Atomen derivatisiert ist, oder ein Phenylring ist, der gegebenenfalls mit Alkylgruppen, die bis zu 4 C-Atome enthalten, und/oder mit bis zu drei Gruppen der Struktur O-X, in der O Sauerstoff und X Wasserstoff, Methyl, Ethyl, n-Propyl, i-Propyl oder tert.-Butyl ist, und/oder mit einer Gruppe der Struktur -COOY, in der Y Wasserstoff, Methyl, Ethyl, n-Propyl, i-Propyl, tert.-Butyl, i-Butyl, i-Pentyl oder neo-Pentyl ist, derivatisiert ist, oder ein Chromansystem ist, das vorzugsweise mit bis zu 5 Alkylgruppen, die bis zu 3 C-Atome enthalten, derivatisiert ist,

mit einer alpha-Amino-Verbindung der Formel III, worin

$R^1$ eine Gruppe der Struktur A-B ist, wobei A = $-(CH_2)_n$ und n = 1-4 und B eine Säurefunktion ist, ausgewählt aus der Gruppe Carboxylfunktion, die gegebenenfalls verestert sein kann oder als Amid vorliegt, wobei die Ester einen Alkohol mit bis zu 17 C-Atomen enthalten, Sulfonsäurefunktion, eine Funktion einer Säure des Phosphors, eine Boronsäurefunktion und Tetrazolgruppen, oder $R^1$ eine Gruppe der Struktur A-B-C ist, wobei A obige Bedeutung hat, B Carbonyl oder Sulfonyl ist und die Gruppe C sich von einer N-gebundenen alpha, beta, gamma oder delta Aminosäure oder der Gruppe der N-glycosidisch verknüpften Uronsäuren ableitet, und

$R^5$ H,$(C_1-C_4)$-Alkyl-, Allyl-, Benzyl- oder p-Methoxybenzylgruppe,

wobei falls erforderlich die reaktiven Hydroxy-, Amino- oder Carbonylgruppen mit in der Peptidchemie üblichen Schutzgruppen geschützt sind, bedeutet, in der Gegenwart einer Alkalilauge, eines Alkalicarbonates oder einer organischen Base in Wasser oder einem polaren Lösungsmittel, bevorzugt $(C_1-C_4)$-Alkohol, DMF oder Acetonitril zu einer Verbindung der Formel IV, wobei die Reste ihre vorher genannte Bedeutung beibehalten, umsetzt, in dem Produkt selektiv den Rest $R^5$ hydrolytisch mit HCl/Eisessig oder Trifluoressigsäure/Dichlormethan oder hydrogenolytisch in Gegenwart von Palladium/Kohle in einem $(C_1-C_4)$-Alkohol, Essigsäure oder Ethylacetat als Lösungsmittel abspaltet, und das entstandene Produkt, worin $R^5$ ein Wasserstoffatom ist, mit einem Phenylalaninderivat der Formel V, worin

$R^2$ und $R^3$ gleich oder verschieden sein können und Alkylgruppen mit bis zu 4 C-Atomen sind oder zusammen einen heterocyclischen Ring mit bis zu 8 Ringgliedern bilden, der mit einer Hydroxygruppe oder einer Hydroxyalkylgruppe mit bis zu 3 C-Atomen derivatisiert sein kann und diese Hydroxygruppe gegebenenfalls verestert vorliegt, wobei die entsprechenden Säuren Carbonsäuren sind, die bis zu 17 C-Atome enthalten, und in

der das mit * gezeichnete C-Atom in der R oder S Struktur, vorzugsweise aber in der R-Struktur vorliegt, bedeutet nach einem in der Peptidchemie üblichen Kondensationsverfahren zu einer geschützten Verbindung der Formel VI, wobei die Reste ihrer vorhergenannte Bedeutung beibehalten, umsetzt, dann in dem erhaltenen Produkt die am Phenylalanylrest gebundene Cyanogruppe nach einem bekannten dreistufigen Verfahren

a) Umsetzung mit Schwefelwasserstoff/Triethylamin zu Thioamid
b) Umsetzung des Thioamids mit Methyliodid in Aceton zu Methylthioimid und
c) Umsetzung des Methylthioimides mit Ammoniumacetat in Methanol

zu einem Amidinoderivat der Formel I umsetzt und falls erforderlich, die Schutzgruppe nach in der Peptidchemie üblichen Verfahren abspaltet, wobei eine weitere Verbindung der Formel I entsteht, und anschließend die Verbindung der Formel I in ein anderes pharmakologisch verträgliches Salz überführt.

**16.** Diagnostisches oder therapeutisches Mittel enthaltend eine Verbindung nach Anspruch 1.

**17.** Verwendung einer Verbindung nach Anspruch 1 in einem Verfahren zur Herstellung eines Diagnostikums oder eines Arzneimittels mit antithrombotischer Wirkung.

**Patentansprüche für folgende Vertragsstaaten : ES, GR**

**1.** Verfahren zur Herstellung einer Verbindung der Formel I

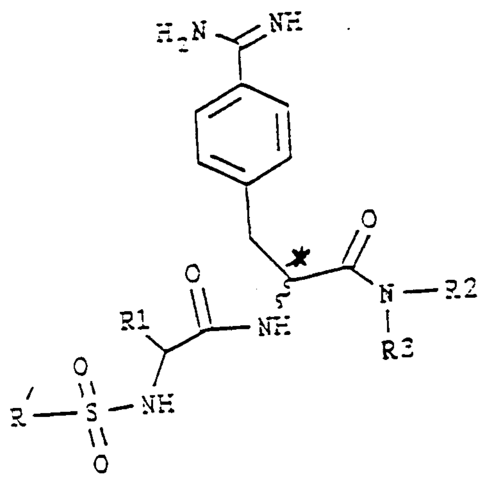

I

worin

R' ein in der alpha- oder beta-Position gebundener Naphthalinring ist, der gegebenenfalls mit Alkylgruppen, die bis zu 3 C-Atome enthalten, und/oder Alkoxygruppen mit jeweils bis zu 3 C-Atomen, derivatisiert ist, oder ein in der alpha- oder beta-Position gebundener Tetralinring oder Indanring ist, der gegebenenfalls mit Alkylgruppen, die aus bis zu 3 C-Atomen bestehen, und/oder auch Alkoxygruppen mit jeweils bis zu 3 C-Atomen derivatisiert ist,
oder ein Phenylring ist, der gegebenenfalls mit Alkylgruppen, die bis zu 4 C-Atome enthalten, und/oder mit bis zu drei Gruppen der Struktur O-X, in der O Sauerstoff und X Wasserstoff, Methyl, Ethyl, n-Propyl, i-Propyl oder tert.-Butyl ist, und/oder mit einer Gruppe der Struktur -COOY, in der Y Wasserstoff, Methyl, Ethyl, n-Propyl, i-Propyl, tert.-Butyl, i-Butyl, i-Pentyl oder neo-Pentyl ist, derivatisiert ist,
oder ein Chromansystem ist, das vorzugsweise mit bis zu 5 Alkylgruppen, die bis zu 3 C-Atome enthalten, derivatisiert ist,

$R_1$ eine Gruppe der Struktur A-B ist, wobei A = $-(CH_2)_n-$ und n = 1-4 und B eine Säurefunktion ist, ausgewählt aus der Gruppe Carboxylfunktion, die gegebenenfalls verestert sein kann oder als Amid vorliegt, wobei die Ester einen Alkohol mit bis zu 17 C-Atomen enthalten, Sulfonsäurefunktion, eine Funktion einer Säure des Phosphors,

eine Boronsäurefunktion und Tetrazolgruppe, oder $R_1$ eine Gruppe der Struktur A-B-C ist, wobei A obige Bedeutung hat, B Carbonyl oder Sulfonyl ist, und die Gruppe C sich von einer N-gebundenen alpha, beta, gamma oder delta Aminosäure oder der Gruppe der N-glycosidisch verknüpften Uronsäuren ableitet, und

$R_2$ und $R_3$ gleich oder verschieden sein können und Alkylgruppen mit bis zu 4 C-Atomen sind oder zusammen einen heterocyclischen Ring mit bis zu 8 Ringgliedern bilden, der mit einer Hydroxygruppe oder einer Hydroxyalkygruppe mit bis zu 3 C-Atomen derivatisiert sein kann und diese Hydroxygruppe gegebenenfalls verestert vorliegt, wobei die entsprechenden Säuren Carbonsäuren sind, die bis zu 17 C-Atome enthalten, und in der das mit * gezeichnete C-Atom in der R oder S Struktur, vorzugsweise aber in der R-Struktur vorliegt,

dadurch gekennzeichnet, daß man eine Verbindung der Formel II

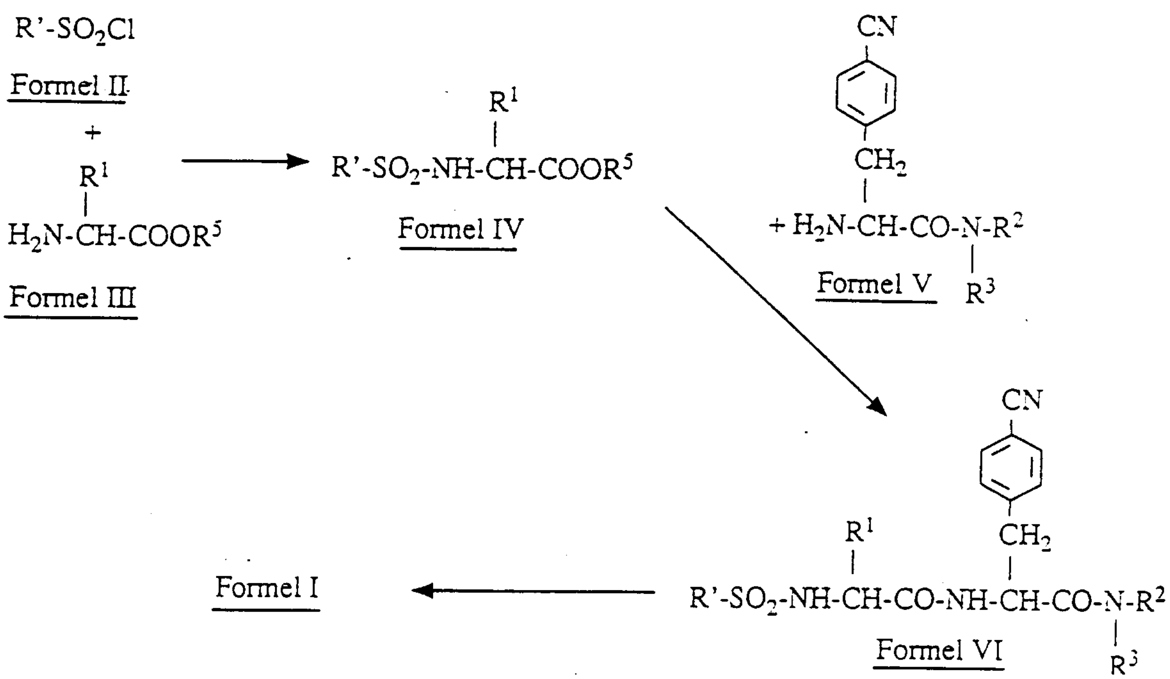

Formel II

Formel III

Formel IV

Formel V

Formel VI

Formel I

worin

R' ein in der alpha- oder beta-Position gebundener Naphthalinring ist, der gegebenenfalls mit Alkylgruppen, die bis zu 3 C-Atome enthalten, und/oder Alkoxygruppen mit jeweils bis zu 3 C-Atomen, derivatisiert ist, oder ein in der alpha- oder beta-Position gebundener Tetralinring oder Indanring ist, der gegebenenfalls mit Alkylgruppen, die aus bis zu 3 C-Atomen bestehen, und/oder auch Alkoxygruppen mit jeweils bis zu 3 C-Atomen derivatisiert ist, oder ein Phenylring ist, der gegebenenfalls mit Alkylgruppen, die bis zu 4 C-Atome enthalten, und/oder mit bis zu drei Gruppen der Struktur O-X, in der O Sauerstoff und X Wasserstoff, Methyl, Ethyl, n-Propyl, i-Propyl oder tert.-Butyl ist, und/oder mit einer Gruppe der Struktur -COOY, in der Y Wasserstoff, Methyl, Ethyl, n-Propyl, i-Propyl, tert.-Butyl, i-Butyl, i-Pentyl oder neo-Pentyl ist, derivatisiert ist, oder ein Chromansystem ist, das vorzugsweise mit bis zu 5 Alkylgruppen, die bis zu 3 C-Atome enthalten, derivatisiert ist,

mit einer alpha-Amino-Verbindung der Formel III, worin

$R^1$ eine Gruppe der Struktur A-B ist, wobei A = $-(CH_2)_n$ und n = 1-4 und B eine Säurefunktion ist, ausgewählt aus der Gruppe Carboxylfunktion, die gegebenenfalls verestert sein kann oder als Amid vorliegt, wobei die Ester einen Alkohol mit bis zu 17 C-Atomen enthalten, Sulfonsäurefunktion, eine Funktion einer Säure des Phosphors, eine Boronsäurefunktion und Tetrazolgruppen, oder $R^1$ eine Gruppe der Struktur A-B-C ist, wobei A obige Bedeutung hat, B Carbonyl oder Sulfonyl ist und die Gruppe C sich von einer N-gebundenen alpha, beta, gamma oder delta Aminosäure oder der Gruppe der N-glycosidisch verknüpften Uronsäuren ableitet, und

$R^5$ H,($C_1$-$C_4$)-Alkyl-, Allyl-, Benzyl- oder p-Methoxybenzylgruppe,

wobei falls erforderlich die reaktiven Hydroxy-, Amino- oder Carbonylgruppen mit in der Peptidchemie üblichen Schutzgruppen geschützt sind, bedeutet, in der Gegenwart einer Alkalilauge, eines Alkalicarbonates oder einer organischen Base in Wasser oder einem polaren Lösungsmittel, bevorzugt ($C_1$-$C_4$)-Alkohol, DMF oder Acetonitril zu einer Verbindung der Formel IV, wobei die Reste ihre vorher genannte Bedeutung beibehalten, umsetzt, in dem Produkt selektiv den Rest $R^5$ hydrolytisch mit HCl/Eisessig oder Trifluoressigsäure/Dichlormethan oder hydrogenolytisch in Gegenwart von Palladium/Kohle in einem ($C_1$-$C_4$)-Alkohol, Essigsäure oder Ethylacetat als Lösungsmittel abspaltet, und das entstandene Produkt, worin $R^5$ ein Wasserstoffatom ist, mit einem Phenylalaninderivat der Formel V, worin

$R^2$ und $R^3$ gleich oder verschieden sein können und Alkylgruppen mit bis zu 4 C-Atomen sind oder zusammen einen heterocyclischen Ring mit bis zu 8 Ringgliedern bilden, der mit einer Hydroxygruppe oder einer Hydroxyalkylgruppe mit bis zu 3 C-Atomen derivatisiert sein kann und diese Hydroxygruppe gegebenenfalls verestert vorliegt, wobei die entsprechenden Säuren Carbonsäuren sind, die bis zu 17 C-Atome enthalten, und in der das mit * gezeichnete C-Atom in der R oder S Struktur, vorzugsweise aber in der R-Struktur vorliegt,

bedeutet nach einem in der Peptidchemie üblichen Kondensationsverfahren zu einer geschützten Verbindung der Formel VI, wobei die Reste ihrer vorhergenannte Bedeutung beibehalten, umsetzt, dann in dem erhaltenen Produkt die am Phenylalanylrest gebundene Cyanogruppe nach einem bekannten dreistufigen Verfahren

a) Umsetzung mit Schwefelwasserstoff/Triethylamin zu Thioamid
b) Umsetzung des Thioamids mit Methyliodid in Aceton zu Methylthioimid und
c) Umsetzung des Methylthioimides mit Ammoniumacetat in Methanol

zu einem Amidinoderivat der Formel I umsetzt und falls erforderlich, die Schutzgruppe nach in der Peptidchemie üblichen Verfahren abspaltet, wobei eine weitere Verbindung der Formel I entsteht, und anschließend die Verbindung der Formel I in ein anderes pharmakologisch verträgliches Salz überführt.

**2.** Verfahren nach Anspruch 1, dadurch gekennzeichnet, daß eine Verbindung hergestellt wird, in der das mit * gekennzeichnete C-Atom in der R-Struktur vorliegt.

**3.** Verfahren nach Anspruch 1, dadurch gekennzeichnet, daß eine Verbindung hergestellt wird, in der R' $\beta$-Naphthyl, R1 -CH2-COOX mit X gleich Wasserstoff und R2 und R3 zusammen Piperidin sind.

**4.** Verfahren nach Anspruch 1, dadurch gekennzeichnet, daß eine Verbindung hergestellt wird, in der R' $\beta$-6,7-Dimethoxynaphthyl-, R1 -CH2-COOX mit X gleich Wasserstoff und R2 und R3 zusammen Piperidin sind.

**5.** Verfahren nach Anspruch 1, dadurch gekennzeichnet, daß eine Verbindung hergestellt wird, in der R' $\beta$-Tetralin, R1 -CH2-COOX mit X gleich Wasserstoff und R2 und R3 zusammen Piperidin sind.

**6.** Verfahren nach Anspruch 1, dadurch gekennzeichnet, daß eine Verbindung hergestellt wird, in der R' 4-Methoxy-2,3,6-trimethylphenyl-, R1 -CH2-COOX mit X gleich Wasserstoff und R2 und R3 zusammen Piperidin sind.

**7.** Verfahren nach Anspruch 1, dadurch gekennzeichnet, daß eine Verbindung hergestellt wird, in der R' 4-Carboxyphenyl-, R1 -CH2-COOX mit X gleich Wasserstoff und R2 und R3 zusammen Piperidin sind.

**8.** Verfahren nach Anspruch 1, dadurch gekennzeichnet, daß eine Verbindung hergestellt wird, in der R' 4-Hydroxy-2,3,6,-trimethylphenyl-, R1 -CH2-COOX mit X gleich Wasserstoff und R2 und R3 zusammen Piperidin sind.

**9.** Verfahren nach Anspruch 1, dadurch gekennzeichnet, daß eine Verbindung hergestellt wird, in der R' 2,2,5,7,8,-Pentamethylchroman-, R1 -CH2-COOX mit X gleich Wasserstoff und R2 und R3 zusammen Piperidin sind.

**10.** Verfahren nach einem der Ansprüche 1-8, dadurch gekennzeichnet, daß eine Verbindung hergestellt wird, in der X einen Alkoholrest darstellt, der bis zu 17 C-Atome aufweist.

**11.** Verfahren nach einem der Ansprüche 1-8, dadurch gekennzeichnet, daß eine Verbindung hergestellt wird, in der $R_1$ die Struktur -(CH2)n-SO3H mit n = 1 bis 4 aufweist.

**12.** Verfahren nach einem der Ansprüche 1-8, dadurch gekennzeichnet, daß eine Verbindung hergestellt wird, in der $R_1$ die Struktur -(CH2)n-PO(OH)2 mit n = 1 bis 4 aufweist.

**13.** Verfahren nach einem der Ansprüche 1-10, dadurch gekennzeichnet, daß eine Verbindung hergestellt wird, in der R2 und R3 zusammen 3-Hydroxymethylpiperidin ist.

**14.** Verfahren nach Anspruch 13, dadurch gekennzeichnet, daß eine Verbindung hergestellt wird, in der die Hydroxyfunktion mit einer Carbonsäure verestert ist, die bis zu 17 C-Atome enthält.

**Claims**

**Claims for the following Contracting States : AT, BE, CH, DE, DK, FR, GB, IT, LI, LU, NL, PT, SE**

**1.** A compound of the formula I

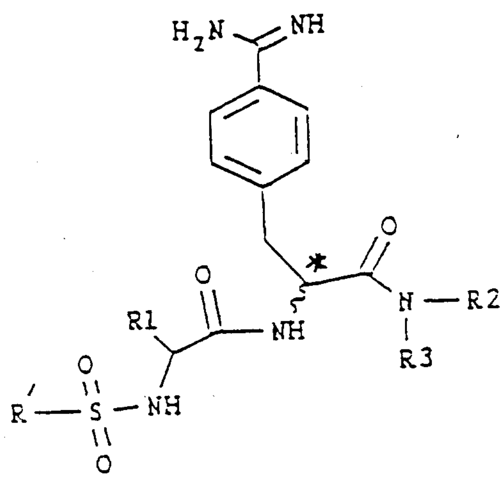

I

in which

| | |
|---|---|
| R' | is a naphthalene ring which is bonded in the alpha or beta position and is optionally derivatized with alkyl groups which contain up to 3 carbon atoms, and/or alkoxy groups each with up to 3 carbon atoms, or is a tetralin ring or indane ring which is bonded in the alpha or beta position and is optionally derivatized with alkyl groups which are composed of up to 3 carbon atoms, and/or also alkoxy groups each with up to 3 carbon atoms,<br>or is a phenyl ring which is optionally derivatized with alkyl groups which contain up to 4 carbon atoms, and/or with up to three groups of the structure O-X in which O is oxygen and X is hydrogen, methyl, ethyl, n-propyl, i-propyl or tert.-butyl, and/or with a group of the structure -COOY in which Y is hydrogen, methyl, ethyl, n-propyl, i-propyl, tert.-butyl, i-butyl, i-pentyl or neo-pentyl,<br>or is a chroman system which is preferably derivatized with up to 5 alkyl groups which contain up to 3 carbon atoms, |
| $R_1$ | is a group of the structure A-B with A = $-(CH_2)_n-$and n = 1-4 and B is an acid functionality selected from the group comprising carboxyl functionality which optionally can be esterified or is in amide form, the esters containing an alcohol with up to 17 carbon atoms, sulfonic acid functionality, a functionality of an acid of phosphorus, a boronic acid functionality and tetrazole group, or $R_1$ is a group of the structure A-B-C where A has the above meaning, B is carbonyl or sulfonyl and the group C is derived from an N-bonded alpha, beta, gamma or delta amino acid or from the group of N-glycosidically linked uronic acids, and |
| $R_2$ and $R_3$ | can be identical or different and are alkyl groups with up to 4 carbon atoms or together form a heterocyclic ring which has up to 8 ring members and which can be |

derivatized with a hydroxyl group or a hydroxyalkyl group with up to 3 carbon atoms, and this hydroxyl group is optionally in esterified form, the corresponding acids being carboxylic acids which contain up to 17 carbon atoms, and in which the carbon atom marked with * is in the R or S structure, but preferably in the R structure.

**2.** A compound as claimed in claim 1, wherein the carbon atom identified by * is in the R structure.

**3.** A compound as claimed in claim 1, in which R' is $\beta$-naphthyl, $R_1$ is $-CH_2-COOX$ with X equal to hydrogen and $R_2$ and $R_3$ together are piperidine.

**4.** A compound as claimed in claim 1, in which R' is $\beta$-6,7-dimethoxynaphthyl, $R_1$ is $-CH_2-COOX$ with X equal to hydrogen and $R_2$ and $R_3$ together are piperidine.

**5.** A compound as claimed in claim 1, in which R' is $\beta$-tetralin, $R_1$ is $-CH_2-COOX$ with X equal to hydrogen and $R_2$ and $R_3$ together are piperidine.

**6.** A compound as claimed in claim 1, in which R' is 4-methoxy-2,3,6-trimethylphenyl, $R_1$ is $-CH_2-COOX$ with X equal to hydrogen and $R_2$ and $R_3$ together are piperidine.

**7.** A compound as claimed in claim 1, in which R' is 4-carboxyphenyl, $R_1$ is $-CH_2-COOX$ with X equal to hydrogen and $R_2$ and $R_3$ together are piperidine.

**8.** A compound as claimed in claim 1, in which R' is 4-hydroxy-2,3,6-trimethylphenyl, $R_1$ is $-CH_2-COOX$ with X equal to hydrogen and $R_2$ and $R_3$ together are piperidine.

**9.** A compound as claimed in claim 1, in which R' is 2,2,5,7,8-pentamethylchroman, $R_1$ is $-CH_2-COOX$ with X equal to hydrogen and $R_2$ and $R_3$ together are piperidine.

**10.** A compound as claimed in any of claims 1-8, wherein X is an alcohol residue which has up to 17 carbon atoms.

**11.** A compound as claimed in any of claims 1-8, where $R_1$ has the structure $-(CH_2)_n-SO_3H$ with n = 1 to 4.

**12.** A compound as claimed in any of claims 1-8, where $R_1$ has the structure $-(CH_2)_n-PO(OH)_2$ with n = 1 to 4.

**13.** A compound as claimed in any of claims 1-10, wherein $R_2$ and $R_3$ together are 3-hydroxymethyl-piperidine.

**14.** A compound as claimed in claim 13, wherein the hydroxyl functionality is esterified with a carboxylicacid which contains up to 17 carbon atoms.

**15.** A process for the preparation of a compound of the formula I in claim 1, which comprises reaction of a compound of the formula II

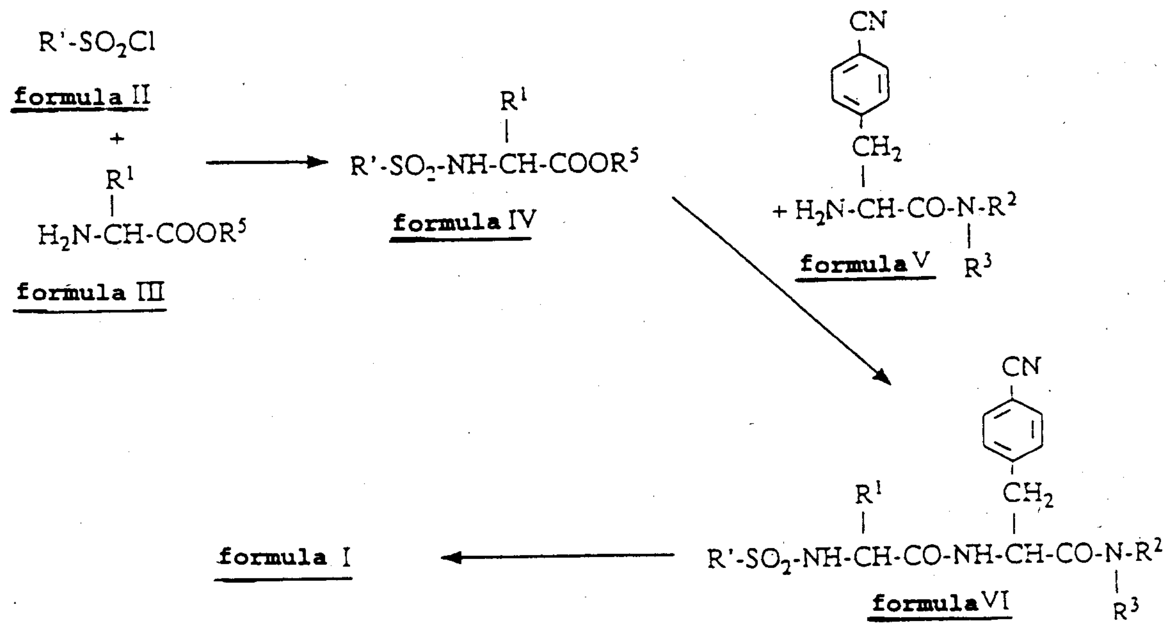

in which

R' is a naphthalene ring which is bonded in the alpha or beta position and is optionally derivatized with alkyl groups which contain up to 3 carbon atoms, and/or alkoxy groups each with up to 3 carbon atoms, or is a tetralin ring or indane ring which is bonded in the alpha oar beta position and is optionally derivatized with alkyl groups which are composed of up to 3 carbon atoms, and/or also alkoxy groups each with up to 3 carbon atoms, or is a phenyl ring which is optionally derivatized with alkyl groups which contain up to 4 carbon atoms, and/or with up to three groups of the structure O-X in which O is oxygen and X is hydrogen, methyl, ethyl, n-propyl, i-propyl or tert.-butyl, and/or with a group of the structure -COOY in which Y is hydrogen, methyl, ethyl, n-propyl, i-propyl, tert.-butyl, i-butyl, i-pentyl or neo-pentyl, or is a chroman system which is preferably derivatized with up to 5 alkyl groups which contain up to 3 carbon atoms,

with an alpha-amino compound of the formula III

in which

$R^1$ is a group of the structure A-B with A = $-(CH_2)_n-$and n = 1-4 and B is an acid functionality selected from the group comprising carboxyl functionality which optionally can be esterified or is in amide form, the esters containing an alcohol with up to 17 carbon atoms, sulfonic acid functionality, a functionality of an acid of phosphorus, a boronic acid functionality and tetrazole group, for $R^1$ is a group of the structure A-B-C where A has the above meaning, B is carbonyl or sulfonyl and the group C is derived from an N-bonded alpha, beta, gamma or delta amino acid or from the group of N-glycosidically linked uronic acids, and

$R^5$ is H, $(C_1\text{-}C_4)$-alkyl, allyl, benzyl or p-methoxybenzyl group,

where, if necessary, the reactive hydroxyl, amino or carbonyl groups are protected by protective groups customary in peptide chemistry, in the presence of an alkali metal hydroxide solution, of an alkali metal carbonate or of an organic base in water or a polar solvent, preferably $(C_1\text{-}C_4)$-alcohol, DMF or acetonitrile, to give a compound of the formula IV where the radicals retain their aforementioned meaning, selective elimination of the radical $R^5$ in the product by hydrolysis with HCl/glacial acetic acid or trifluoroacetic acid/dichloromethane or by hydrogenolysis in the presence of palladium/carbon in a $(C_1\text{-}C_4)$-alcohol, acetic acid or ethyl acetate as solvent, and reaction of the resulting product in which $R^5$ is a hydrogen atom with a phenylalanine derivative of the formula V in which

$R^2$ and $R^3$ can be identical or different and are alkyl groups with up to 4 carbon atoms or together form a heterocyclic ring which has up to 8 ring members and which can be derivatized with a hydroxyl group or a hydroxyalkyl group with up to 3 carbon atoms, and this hydroxyl group is optionally in esterified form, the corresponding acids being carboxylic acids which contain up to 17 carbon atoms, and in which the carbon atom marked with * is in the R or S structure, but preferably in the R structure by a

condensation process customary in peptide chemistry to give a protected compound of the formula VI where the radicals retain their aforementioned meaning, then conversion of the cyano group bonded to the phenylalanyl radical in the resulting product in a known three-stage process

a) conversion with hydrogen sulfide/triethylamine into thioamide

b) conversion of the thioamide with methyl iodide in acetone into methylthioimide and

c) reaction of the methylthioimide with ammonium acetate in methanol

into an amidino derivative of the formula I and, if necessary, elimination of the protective group by processes customary in peptide chemistry, resulting in another compound of the formula I, and subsequent conversion of the compound of the formula I into another pharmacologically suitable salt.

**16.** A diagnostic or therapeutic composition containing a compound as claimed in claim 1.

**17.** The use of a compound as claimed in claim 1 in a process for the production of a diagnostic aid or of a pharmaceutical with antithrombotic action.

**Claims for the following Contracting States : ES, GR**

**1.** A process for the preparation of a compound of the formula I

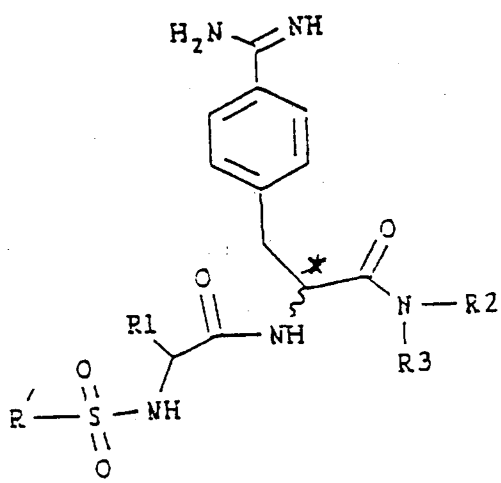

I

in which

R' is a naphthalene ring which is bonded in the alpha or beta position and is optionally derivatized with alkyl groups which contain up to 3 carbon atoms, and/or alkoxy groups each with up to 3 carbon atoms, or is a tetralin ring or indane ring which is bonded in the alpha or beta position and is optionally derivatized with alkyl groups which are composed of up to 3 carbon atoms, and/or also alkoxy groups each with up to 3 carbon atoms,

or is a phenyl ring which is optionally derivatized with alkyl groups which contain up to 4 carbon atoms, and/or with up to three groups of the structure O-X in which O is oxygen and X is hydrogen, methyl, ethyl, n-propyl, i-propyl or tert.-butyl, and/or with a group of the structure -COOY in which Y is hydrogen, methyl, ethyl, n-propyl, i-propyl, tert.-butyl, i-butyl, i-pentyl or neo-pentyl,

or is a chroman system which is preferably derivatized with up to 5 alkyl groups which contain up to 3 carbon atoms,

$R_1$ is a group of the structure A-B with A = $-(CH_2)_n-$and n = 1-4 and B is an acid functionality selected from the group comprising carboxyl functionality which optionally can be esterified or is in amide form, the esters containing an alcohol with up to 17 carbon atoms, sulfonic acid functionality, a functionality of an acid of phosphorus, a boronic acid functionality and tetrazole group, or $R_1$ is a group of the structure A-B-C where A has the above meaning, B is carbonyl or sulfonyl, and the group C is derived from an N-bonded alpha, beta, gamma or delta amino acid or from the group of N-glycosidically linked uronic acids, and

$R_2$ and $R_3$ can be identical or different and are alkyl groups with up to 4 carbon atoms or together form a heterocyclic ring which has up to 8 ring members and which can be derivatized with a hydroxyl group or a hydroxyalkyl group with up to 3 carbon atoms, and this hydroxyl group is optionally in esterified form, the corresponding acids being carboxylic acids which contain up to 17 carbon atoms, and in which the carbon atom marked with * is in the R or S structure, but preferably in the R structure which comprises reaction of a compound of the formula II

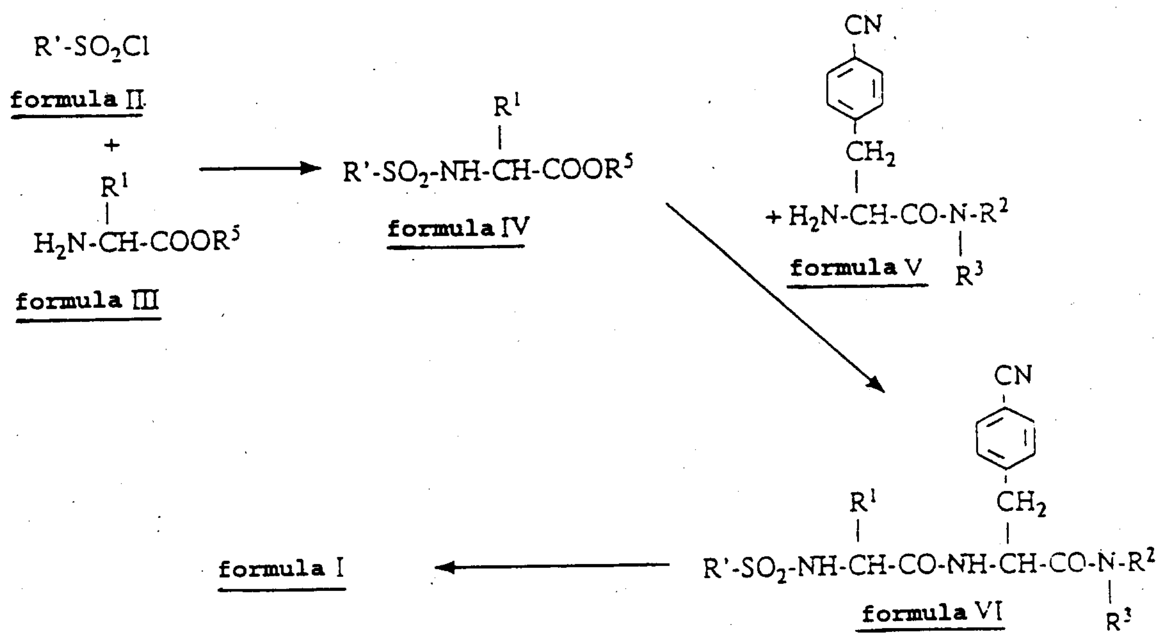


in which

R' is a naphthalene ring which is bonded in the alpha or beta position and is optionally derivatized with alkyl groups which contain up to 3 carbon atoms, and/or alkoxy groups each with up to 3 carbon atoms, or is a tetralin ring or indane ring which is bonded in the alpha or beta position and is optionally derivatized with alkyl groups which are composed of up to 3 carbon atoms, and/or also alkoxy groups each with up to 3 carbon atoms,
or is a phenyl ring which is optionally derivatized with alkyl groups which contain up to 4 carbon atoms, and/or with up to three groups of the structure O-X in which O is oxygen and X is hydrogen, methyl, ethyl, n-propyl, i-propyl or tert.-butyl, and/or with a group of the structure -COOY in which Y is hydrogen, methyl, ethyl, n-propyl, i-propyl, tert.-butyl, i-butyl, i-pentyl or neo-pentyl,
or is a chroman system which is preferably derivatized with up to 5 alkyl groups which contain up to 3 carbon atoms,

with an alpha-amino compound of the formula III

in which

$R^1$ is a group of the structure A-B with A = $-(CH_2)_n-$and n = 1-4 and B is an acid functionality selected from the group comprising carboxyl functionality which optionally can be esterified or is in amide form, the esters containing an alcohol with up to 17 carbon atoms, sulfonic acid functionality, a functionality of an acid of phosphorus, a boronic acid functionality and tetrazole group, or $R^1$ is a group of the structure A-B-C where A has the above meaning, B is carbonyl or sulfonyl and the group C is derived from an N-bonded alpha, beta, gamma or delta amino acid or from the group of N-glycosidically linked uronic acids, and

$R^5$ is H, $(C_1\text{-}C_4)$-alkyl, allyl, benzyl or p-methoxybenzyl group,

where, if necessary, the reactive hydroxyl, amino or carbonyl groups are protected by protective groups customary in peptide chemistry, in the presence of an alkali metal hydroxide solution, of an alkali metal

carbonate or of an organic base in water or a polar solvent, preferably ($C_1$-$C_4$)-alcohol, DMF or acetonitrile, to give a compound of the formula IV where the radicals retain their aforementioned meaning, selective elimination of the radical $R^5$ in the product by hydrolysis with HCl/glacial acetic acid or trifluoroacetic acid/dichloromethane or by hydrogenolysis in the presence of palladium/carbon in a ($C_1$-$C_4$)-alcohol, acetic acid or ethyl acetate as solvent, and reaction of the resulting product in which $R^5$ is a hydrogen atom with a phenylalanine derivative of the formula V in which

$R^2$ and $R^3$ can be identical or different and are alkyl groups with up to 4 carbon atoms or together form a heterocyclic ring which has up to 8 ring members and which can be derivatized with a hydroxyl group or a hydroxyalkyl group with up to 3 carbon atoms, and this hydroxyl group is optionally in esterified form, the corresponding acids being carboxylic acids which contain up to 17 carbon atoms, and in which the carbon atom marked with * is in the R or S structure, but preferably in the R structure

by a condensation process customary in peptide chemistry to give a protected compound of the formula VI where the radicals retain their aforementioned meaning, then conversion of the cyano group bonded to the phenylalanyl radical in the resulting product in a known three-stage process

a) conversion with hydrogen sulfide/triethylamine into thioamide

b) conversion of the thioamide with methyl iodide in acetone into methylthioimide and

c) reaction of the methylthioimide with ammonium acetate in methanol

into an amidino derivative of the formula I and, if necessary, elimination of the protective group by processes customary in peptide chemistry, resulting in another compound of the formula I, and subsequent conversion of the compound of the formula I into another pharmacologically suitable salt.

**2.** The process as claimed in claim 1, wherein a compound is prepared in which the carbon atom identified by * is in the R structure.

**3.** The process as claimed in claim 1, wherein a compound is prepared in which R' is $\beta$-naphthyl, $R_1$ is $-CH_2-COOX$ with X equal to hydrogen and $R_2$ and $R_3$ together are piperidine.

**4.** The process as claimed in claim 1, wherein a compound is prepared in which R' is $\beta$-6,7-dimethoxynaphthyl, $R_1$ is $-CH_2-COOX$ with X equal to hydrogen and $R_2$ and $R_3$ together are piperidine.

**5.** The process as claimed in claim 1, wherein a compound is prepared in which R' is $\beta$-tetralin, $R_1$ is $-CH_2-COOX$ with X equal to hydrogen and $R_2$ and $R_3$ together are piperidine.

**6.** The process as claimed in claim 1, wherein a compound is prepared in which R' is 4-methoxy-2,3,6-trimethylphenyl, $R_1$ is $-CH_2-COOX$ with X equal to hydrogen and $R_2$ and $R_3$ together are piperidine.

**7.** The process as claimed in claim 1, wherein a compound is prepared in which R' is 4-carboxyphenyl, $R_1$ is $-CH_2-COOX$ with X equal to hydrogen and $R_2$ and $R_3$ together are piperidine.

**8.** The process as claimed in claim 1, wherein a compound is prepared in which R' is 4-hydroxy-2,3,6-trimethylphenyl, $R_1$ is $-CH_2-COOX$ with X equal to hydrogen and $R_2$ and $R_3$ together are piperidine.

**9.** The process as claimed in claim 1, wherein a compound is prepared in which R' is 2,2,5,7,8-pentamethylchroman, $R_1$ is $-CH_2-COOX$ with X equal to hydrogen and $R_2$ and $R_3$ together are piperidine.

**10.** The process as claimed in any of claims 1-8, wherein a compound is prepared in which X is an alcohol residue which has up to 17 carbon atoms.

**11.** The process as claimed in any of claims 1-8, wherein a compound is prepared in which $R_1$ has the structure $-(CH_2)_n-SO_3H$ with n = 1 to 4.

**12.** The process as claimed in any of claims 1-8, wherein a compound is prepared in which $R_1$ has the structure $-(CH_2)_n-PO(OH)_2$ with n = 1 to 4.

**13.** The process as claimed in any of claims 1-10, wherein a compound is prepared in which $R_2$ and $R_3$ together are 3-hydroxymethylpiperidine.

**14.** The process as claimed in claim 13, wherein a compound is prepared in which the hydroxyl functionality is esterified with a carboxylic acid which contains up to 17 carbon atoms.

**Revendications**

**Revendications pour les Etats contractants suivants : AT, BE, CH, DE, DK, FR, GB, IT, LI, LU, NL, PT, SE**

**1.** Composé de formule I :

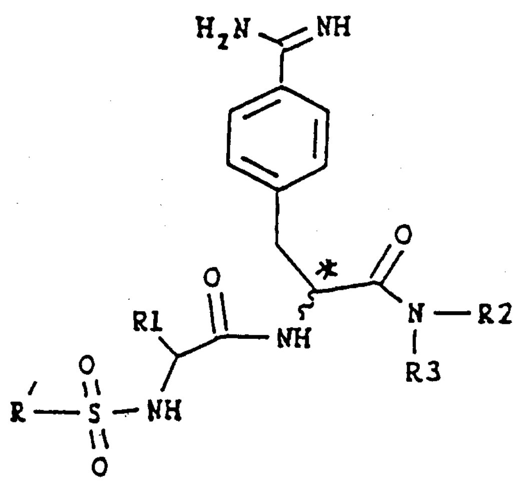

I

dans laquelle

R' est un noyau naphtalénique lié en position alpha ou béta, qui est éventuellement substitué par des groupes alkyles qui contiennent jusqu'à 3 atomes de carbone et/ou avec des groupes alcoxy qui contiennent chacun jusqu'à 3 atomes de carbone, ou un noyau de tétraline ou d'indane lié en position alpha ou bêta, qui est éventuellement substitué par des groupes alkyles qui contiennent jusqu'à 3 atomes de carbone et/ou aussi des groupes alcoxy qui contiennent chacun jusqu'à 3 atomes de carbone,
ou un noyau phénlylique qui est éventuellement substitué par des groupes alkyles qui contiennent jusqu'à 4 atomes de carbone, et/ou par jusqu'à trois groupes ayant la structure O-X, où O est l'oxygène et X est l'hydrogène ou un groupe méthyle, éthyle, n-propyle, i-propyle ou tert-butyle, et/ou avec un groupe ayant la structure -COOY, où Y est l'hydrogène ou un groupe méthyle, éthyle, n-propyle, i-propyle, tert-butyle, i-butyle, i-pentyle ou néopentyle,
ou un système du type chromane qui est substitué, de préférence, par jusqu'à 5 groupes alkyles qui contiennent jusqu'à 3 atomes de carbone,

$R_1$ est un groupe ayant la structure A-B, où A = $-(CH_2)_n-$ et n - 1-4, et B est une fonction acide choisie dans l'ensemble constitué par une fonction carboxyle qui peut éventuellement être estérifiée ou se présente sous la forme d'un amide, les esters contenant un alcool avec jusqu'à 17 atomes de carbone, une fonction acide sulfonique, une fonction d'un acide du phosphore, une fonction acide borique et un groupe tétrazole, ou $R_1$ est un groupe ayant la structure A-B-C, où A a la signification indiquée ci-dessus, B est un groupe carbonyle ou sulfonyle, et le groupe C est dérivé d'un amino-acide alpha, bêta, gamma ou delta lie par un atome d'azote, ou du groupe des acides uroniques liés par une liaison N-glucosidique, et

$R_2$ et $R_3$ peuvent être identiques ou différents et signifient des groupes alkyles contenant jusqu'à 4 atomes de carbone ou forment ensemble un composé hétérocyclique contenant jusqu'à 8 chaînons, qui peut être substitué par un groupe hydroxy ou un groupe hydroxyalkyle contenant jusqu'à 3 atomes de carbone, et ce groupe hydroxy est sous une forme éventuellement estérifiée, les acides, correspondants étant des acides carboxyliques qui contiennent jusqu'à 17 atomes de carbone, et l'atome de carbone

caractérisé par * dans la formule a la structure R ou S, de préférence la structure R.

2. Composé selon la revendication 1, caractérisé en ce que l'atome de carbone caractérisé par * possède la structure R.

3. Composé selon la revendication 1, dans lequel R' est un groupe $\beta$-naphtyle, $R_1$ est un groupe $-CH_2-COOX$ où X signifie l'hydrogène, et $R_2$ et $R_3$ ensemble forment une pipéridine.

4. Composé selon la revendication 1, dans lequel R' est un groupe $\beta$-6,7-diméthoxynaphtyle, $R_1$ est un groupe $-CH_2-COOX$ où X signifie l'hydrogène, et $R_2$ et $R_3$ ensemble forment une pipéridine.

5. Composé seton la revendication 1, dans lequel R' est une $\beta$-tétraline, $R_1$ est un groupe $-CH_2-COOX$ où X signifie l'hydrogène, et $R_2$ et $R_3$ ensemble forment une pipéridine.

6. Composé selon la revendication 1, dans lequel R' est un groupe 4-méthoxy-2,3,6-triméthylphényle, $R_1$ est un groupe $-CH_2-COOX$ où X signifie l'hydrogène, et $R_2$ et $R_3$ ensemble forment une pipéridine.

7. Composé selon la revendication 1, dans lequel R' est un groupe 4-carboxyphényle, $R_1$ est un groupe $-CH_2-COOX$ où X signifie l'hydrogène, et $R_2$ et $R_3$ ensemble forment une pipéridine.

8. Composé selon la revendication 1, dans lequel R' est un groupe 4-hydroxy-2,3,6-triméthylphényle, $R_1$ est un groupe $-CH_2-COOX$ où X signifie l'hydrogène, et $R_2$ et $R_3$ ensemble forment une pipéridine.

9. Composé selon la revendication 1, dans lequel R' est un groupe 2,2,5,7,8-pentaméthylchromane, $R_1$ est un groupe $CH_2-COOX$ où X signifie l'hydrogène, et $R_2$ et $R_3$ ensemble forment une pipéridine.

10. Composé selon l'une des revendications 1 à 8, caractérisé en ce que X représente un reste d'alcool qui comporte jusqu'à 17 atomes de carbone.

11. Composé selon l'une des revendications 1 à 8, dans lequel $R_1$ possède la structure $-(CH_2)_n-SO_3H$ où n = 1 à 4.

12. Composé selon l'une des revendications 1 à 8, dans lequel $R_1$ possède la structure $-(CH_2)_n-PO(OH)_2$ où n = 1 à 4.

13. Composé selon l'une des revendications 1 à 10, caractérisé en ce que $R_2$ et $R_3$ ensemble formant une 3-hydroxyméthylpipéridine.

14. Composé selon la revendication 13, caractérisé en ce que la fonction hydroxy est estérifiée avec un acide carboxylique qui contient jusqu'à 17 atomes de carbone.

15. Procédé de préparation d'un composé de formule I selon la revendication 1, caractérisé en ce qu'on fait réagir un composé de formule II :

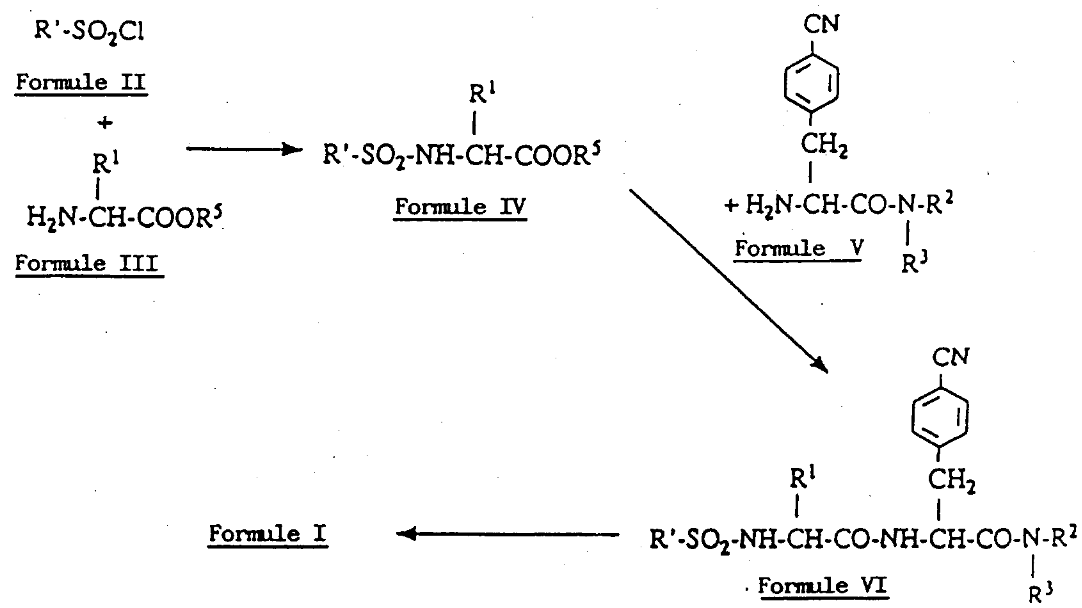

Formule II

Formule III

Formule IV

Formule V

Formule VI

Formule I

où

R' est un noyau naphtalénique lié en position alpha ou bêta, qui est éventuellement substitué par des groupes alkyles qui contiennent jusqu'à 3 atomes de carbone et/ou par des groupes alcoxy qui contiennent chacun jusqu'à 3 atomes de carbone, ou un noyau de tétraline ou d'indane lié en position alpha ou bêta, qui est éventuellement substitué par des groupes alkyles qui contiennent jusqu'à 3 atomes de carbone et/ou aussi des groupes alcoxy qui contiennent chacun jusqu'à 3 atomes de carbone, ou un noyau phénylique qui est éventuellement substitué par des groupes alkyles qui contiennent jusqu'à 4 atomes de carbone, et/ou avec jusqu'à trois groupes ayant la structure O-X, où O est l'oxygène et X est l'hydrogène ou un groupe méthyle, éthyle, n-propyle, i-propyle ou tert-butyle, et/ou avec un groupe ayant la structure -COOY, où Y est l'hydrogène ou un groupe méthyle, éthyle, n-propyle, i-propyle, tert butyle, i-butyle, i-pentyle ou néopentyle, ou un système du type chromane qui est substitué, de préférence, par jusqu'à 5 groupes alkyles qui contiennent jusqu'à 3 atomes de carbone,

avec un composé alpha-amino de formule III, où

$R^1$ est un groupe ayant la structure A-B, où A = $-(CH_2)_n-$ et n = 1-4, et B est une fonction acide choisie dans l'ensemble constitué par une fonction carboxyle qui peut éventuellement être estérifiée ou se présente sous la forme d'un amide, les esters contenant un alcool avec jusqu'à 17 atomes de carbone, une fonction acide sulfonique, une fonction d'un acide du phosphore, une fonction acide borique et des groupes tétrazole, ou $R^1$ est un groupe ayant la structure A-B-C, où A a la signification indiquée ci dessus, B est un groupe carbonyle ou sulfonyle, et le groupe C est dérivé d'un amino-acide alpha, bêta, gamma ou delta lié par un atome d'azote, ou du groupe des acides uroniques liés par une liaison N-glucosidique, et

$R^5$ signifie H ou un groupe alkyle en ($C_1$-$C_4$), allyle, benzyle ou p-méthoxybenzyle,

les groupes réactifs hydroxy, amino ou carbonyle étant protégés, si cela est nécessaire, avec des groupes protecteurs usuels dans la chimie des peptides,

en présence d'une lessive alcaline, d'un carbonate alcalin ou d'une base organique dans de l'eau ou un solvant polaire, de préférence un alcool en ($C_1$-$C_4$), le DMF ou l'acétonitrile, pour obtenir un composé de formule IV dont les restes conservent leur signification indiquée précédemment ; dans le produit obtenu, on sépare sélectivement le reste $R^5$ par hydrolyse avec un mélange de HCl/acide acétique ou d'acide trifluoroacétique/dichlorométhane, ou par hydrogénolyse en présence de palladium/charbon dans un alcool en ($C_1$-$C_4$), l'acide acétique ou l'acétate d'éthyle, comme solvant, et on fait réagir le produit formé, dans lequel $R^5$ est un atome d'hydrogène, avec un dérivé de phénylalanine de formule V, dans laquelle

$R^2$ et $R^3$ peuvent être identiques ou différents et signifient des groupes alkyles contenant jusqu'à 4 atomes de carbone ou forment ensemble un noyau hétérocyclique contenant jusqu'à 8 chaînons, qui peut être substitué par un groupe hydroxy ou un groupe hydroxyalkyle contenant jusqu'à 3 atomes de carbone, et ce groupe hydroxy est sous une forme éventuellement estérifiée, les acides correspondants étant des acides carboxylique s

qui contiennent jusqu'à 17 atomes de carbone, et l'atome de carbone caractérisé par * dans la formule a la structure R ou S, de préférence la structure R,

selon un procédé de condesation usuel dans la chimie des peptides, pour obtenir un composé protégé répondant à la formule VI, dans laquelle les restes conservent leur signification indiquée précédemment,

ensuite, dans le produit obtenu, on transforme le groupe cyano, qui est lié au reste de phénylalanyle, en un dérivé amidino de formule I selon un procédé connu à trois étapes:

a) transformation en thioamide avec un mélange d'acide sulfhydrique/triéthylamine,

b) transformation du thioamide en méthylthioimide avec de l'iodure de méthyle dans de l'acétone, et

c) mise en réaction du méthylthioimide avec de l'acétate d'ammonium dans du méthanol,

et si cela est nécessaire, on sépare le groupe protecteur selon des procédés usuels dans la chimie des peptides, pour former un autre composé de formule I, et ensuite, on transforme le composé de formule I en un autre sel pharmacologiquement acceptable.

**16.** Agent diagnostique ou thérapeutique contenant un composé selon la revendication 1.

**17.** Utilisation d'un composé selon la revendication 1 dans un procédé de préparation d'un agent de diagnostic ou d'un médicament ayant un effet antithrombotique.

**Revendications pour les Etats contractants suivants : ES, GR**

**1.** Procédé de préparation d'un composé de formule I :

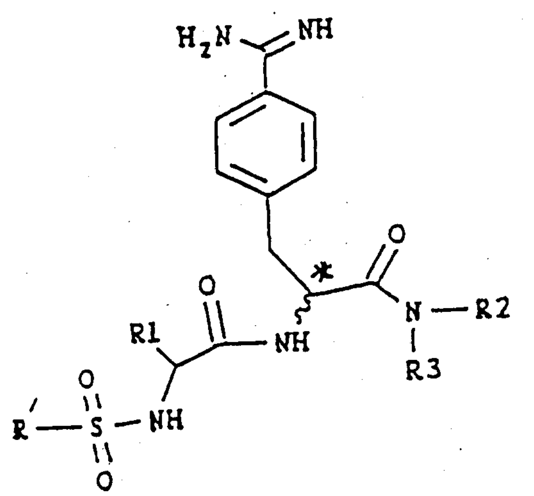

I

dans laquelle

R' est un noyau naphtalénique lié en position alpha ou bêta, qui est éventuellement substitué par des groupes alkyles qui contiennent jusqu'à 3 atomes de carbone et/ou avec des groupes alcoxy qui contiennent chacun jusqu'à 3 atomes de carbone, ou un noyau de tétraline ou d'indane lié en position alpha ou bêta, qui est éventuellement substitué par des groupes alkyles qui contiennent jusqu'à 3 atomes de carbone et/ou aussi des groupes alcoxy qui contiennent chacun jusqu'à 3 atomes de carbone,
ou un noyau phénylique qui est éventuellement substitué par des groupes alkyles qui contiennent jusqu'à 4 atomes de carbone, et/ou par jusqu'à trois groupes ayant la structure O X, où O est l'oxygène et X est l'hydrogène ou un groupe méthyle, éthyle, n-propyle, i-propyle ou tert-butyle, et/ou avec un groupe ayant la structure -COOY, où Y est l'hydrogène ou un groupe méthyle, éthyle, n-propyle, i-propyle, tert-butyle, i-butyle, i-pentyle ou néopentyle,
ou un système du type chromane qui est substitué, de préférence, par jusqu'à 5 groupes alkyles qui contiennent jusqu'à 3 atomes de carbone,

$R_1$ est un groupe ayant la structure A-B, où A = $-(CH_2)_n-$ et n = 1-4, et B est une fonction acide choisie dans l'ensemble constitué par une fonction carboxyle qui peut éventuellement être estérifiée ou se présente sous la forme d'un amide, les esters contenant un

alcool avec jusqu'à 17 atomes de carbone, une fonction acide sulfonique, une fonction d'un acide du phosphore, une fonction acide borique et un groupe tétrazole, ou $R_1$ est un groupe ayant la structure A-B-C, où A a la signification indiquée ci dessus, B est un groupe carbonyle ou sulfonyle, et le groupe C est dérivé d'un amino-acide alpha, bêta, gamma ou delta lié par un atome d'azote, ou du groupe des acides uroniques liés par une liaison N-glucosidique, et

$R_2$ et $R_3$ peuvent être identiques ou différents et signifient des groupes alkyles contenant jusqu'à 4 atomes de carbone ou forment ensemble un composé hétérocyclique contenant jusqu'à 8 chaînons, qui peut être substitué par un groupe hydroxy ou un groupe hydroxyalkyle contenant jusqu'à 3 atomes de carbone, et ce groupe hydroxy est sous une forme éventuellement estérifiée, les acides correspondants étant des acides carboxyliques qui contiennent jusqu'à 17 atomes de carbone, et l'atome de carbone caractérisé par * dans la formule a la structure R ou S, de préférence la structure R,

caractérisé en ce qu'on fait réagir un composé de formule II :

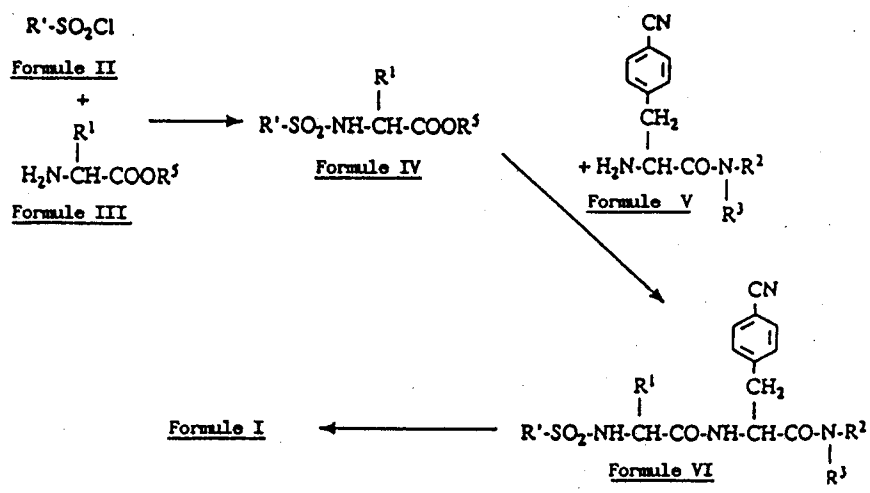

où

R' est un noyau naphtaténique lié en position alpha ou bêta, qui est éventuellement substitué par des groupes alkyles qui contiennent jusqu'à 3 atomes de carbone et/ou par des groupes alcoxy qui contiennent chacun jusqu'à 3 atomes de carbone, ou un noyau de tétraline ou d'indane lié en position alpha ou bêta, qui est éventuellement substitué par des groupes alkyles qui contiennent jusqu'à 3 atomes de carbone et/ou aussi des groupes alcoxy qui contiennent chacun jusqu'à 3 atomes de carbone, ou un noyau phénylique qui est éventuellement substitué par des groupes alkyles qui contiennent jusqu'à 4 atomes de carbone, et/ou avec jusqu'à trois groupes ayant la structure O-X, où O est l'oxygène et X est l'hydrogène ou un groupe méthyle, éthyle, n-propyle, i-propyle ou tert-butyle, et/ou avec un groupe ayant la structure -COOY, où Y est l'hydrogène ou un groupe méthyle, éthyle, n-propyle, i-propyle, tert-butyle, i-butyle, i-pentyle ou néopentyle ou un système du type chromane qui est substitué, de préférence, par jusqu'à 5 groupes alkyles qui contiennent jusqu'à 3 atomes de carbone,

avec un composé alpha-amino de formule III, où

$R^1$ est un groupe ayant la structure A-B, où A - $-(CH_2)_n-$ et n = 1-4, et B est une fonction acide choisie dans l'ensemble constitué par une fonction carboxyle qui peut éventuellement être estérifiée ou se présente sous la forme d'un amide, les esters contenant un alcool avec jusqu'à 17 atomes de carbone, une fonction acide sulfonique, une fonction d'un acide du phosphore, une fonction acide borique et des groupes tétrazole, ou $R^1$ est un groupe ayant la structure A-B-C, où A a la signification indiquée ci dessus, B est un groupe carbonyle ou sulfonyle, et le groupe C est dérivé d'un amino-acide alpha, bêta, gamma ou delta lié par un atome d'azote, ou du groupe des acides uroniques liés par une liaison N-glucosidique, et

$R^5$ signifie H ou un groupe alkyle en ($C_1$-$C_4$), allyle, benzyle ou p-méthoxybenzyle,

les grouper réactifs hydroxy amino ou carbonyle étant protégés, si cela est nécessaire, avec des groupes protecteurs usuels dans la chimie des peptides,

en présence d'une lessive alcaline, d'un carbonate alcalin ou d'une base organique dans de l'eau ou un solvant polaire, de préférence un alcool en ($C_1$-$C_4$), le DMF ou l'acétonitrile, pour obtenir un composé de formule IV dont les restes conservent leur signification indiquée précédemment ; dans le produit obtenu, on sépare sélectivement le reste $R^5$ par hydrolyse avec un mélange de HCl/acide acétique ou d'acide trifluoroacétique/dichlorométhane, ou par hydrogénolyse en présence de palladium/charbon dans un alcool en ($C_1$-$C_4$), l'acide acétique ou l'acétate d'éthyle, comme solvant, et ou fait réagir le produit formé, dans lequel $R^5$ est une atome d'hydrogène, avec un dérivé de phénylalanine de formule V, dans laquelle

$R^2$ et $R^3$ peuvent être identiques ou différents et signifient des groupes alkyles contenant jusqu'à 4 atomes de carbone ou forment ensemble un noyau hétérocyclique contenant jusqu'à 8 chaînons, qui peut être substitué par un groupe hydroxy ou un groupe hydroxyalkyle contenant jusqu'à 3 atomes de carbone, et ce groupe hydroxy est sous une forme éventuellement estérifiée, les acides correspondants étant des acides carboxyliques qui con tiennent jusqu'a 17 atomes de carbone, et l'atome de carbone caractérisé par * dans la formule a la structure R ou S, de préférence la structure R,

selon un procédé de condensation usuel dans la chimie des peptides, pour obtenir un composé protégé répondant à la formule VI, dans laquelle les restes conservent leur signification indiquée précédemment,

ensuite, dans le produit obtenu, on transforme le groupe cyano, qui est lié au reste de phénylalanyle, en un dérivé amidino de formule I selon un procéde connu à trois étapes :

a) transformation en thioamide avec un mélange d'acide sulfhydrique/triéthylamine,
b) transformation du thioamide en méthylthioimide avec de l'iodure de méthyle dans de l'acétone, et
c) mise en réaction du méthylthioimide avec de l'acétate d'ammonium dans du méthanol,

et si cela est nécessaire, on sépare le groupe protecteur selon des procédés usuels dans la chimie des peptides, pour former un autre composé de formule I, et ensuite, on transforme le composé de formule I en un autre sel pharmacologiquement acceptable.

**2.** Procédé selon la revendication 1, caractérisé en ce qu'on prépare un composé dans lequel l'atome de carbone caractérisé par * possède la structure R.

**3.** Procédé selon la revendication 1, caractérisé en ce qu'on prépare un composé dans lequel R' est un groupe $\beta$-naphtyle, $R_1$ est un groupe -$CH_2$ COOX où X signifie l'hydrogène et $R_2$ et $R_3$ ensemble forment une pipéridine.

**4.** Procédé selon la revendication 1, caractérisé en ce qu'on prépare un composé dans lequel R' est un groupe $\beta$-6,7-diméthoxynaphtyle, $R_1$ est un groupe -$CH_2$-COOX où X signifie l'hydrogène et $R_2$ et $R_3$ ensemble forment une pipéridine.

**5.** Procédé selon la revendication 1, caractérisé en ce qu'on prépare un composé dans lequel R' est une $\beta$-tétraline, $R_1$ est un groupe -$CH_2$-COOX où X signifie l'hydrogène, et $R_2$ et $R_3$ ensemble forment une pipéridine.

**6.** Procédé selon la revendication 1, caractérisé en ce qu'on prépare un composé dans lequel R' est un groupe 4-méthoxy-2,3,6-triméthylphényle, $R_1$ est un groupe -$CH_2$-COOX où X signifie l'hydrogène, et $R_2$ et $R_3$ ensemble forment une pipéridine.

**7.** Procédé selon la revendication 1, caractérisé en ce qu'on prépare un composé dans lequel R' est un groupe 4-carboxyphényle, $R_1$ est un groupe -$CH_2$-COOX où X signifie l'hydrogène, et $R_2$ et $R_3$ ensemble forment une pipéridine.

**8.** Procédé selon la revendication 1, caractérisé en ce qu'on prépare un composé dans lequel R' est un groupe 4-hydroxy-2,3,6-triméthylphényle, $R_1$ est un groupe -$CH_2$-COOX où X signifie l'hydrogène, et $R_2$ et $R_3$ ensemble forment une pipéridine.

**9.** Procédé selon la revendication 1, caractérisé en ce qu'on prépare un composé dans lequel R' est un groupe 2,2,5,7,8-pentaméthylchromane, $R_1$ est un groupe -$CH_2$-COOX où X signifie l'hydrogène, et $R_2$ et $R_3$ ensemble forment une pipéridine.

**10.** Procédé selon l'une des revendications 1 à 8, caractérisé en ce qu'on prépare un composé dans lequel X représente un reste d'alcool qui comporte jusqu'à 17 atomes de carbone.

**11.** Procédé selon l'une des revendications 1 à 8, caractérisé en ce qu'on prépare un composé dans lequel $R_1$ présente la structure $-(CH_2)_n-SO_3H$ où n - 1 à 4.

**12.** Procédé selon l'une des revendications 1 à 8, caractérisé en ce qu'on prépare un composé dans lequel $R_1$ présente la structure $-(CH_2)_n-PO(OH)_2$ où n - 1 à 4.

**13.** Procédé selon l'une des revendications 1 à 10, caractérisé en ce qu'on prépare un composé dans lequel $R_2$ et $R_3$ ensemble forment une 3-hydroxyméthylpipéridine.

**14.** Procédé selon la revendication 13, caractérisé en ce qu'on prépare un composé dans lequel la fonction hydroxy est estérifiée avec un acide carboxylique qui contient jusqu'à 17 atomes de carbone.